(19) Europäisches Patentamt
European Patent Office
Office européen des brevets

(11) **EP 4 270 403 A1**

(12) **EUROPEAN PATENT APPLICATION**

(43) Date of publication:
**01.11.2023 Bulletin 2023/44**

(21) Application number: **22382393.1**

(22) Date of filing: **25.04.2022**

(51) International Patent Classification (IPC):
**G16H 10/20** (2018.01)     **G16H 50/20** (2018.01)
**G16H 50/30** (2018.01)     **G06F 16/35** (2019.01)
**G06F 40/30** (2020.01)     G06K 9/62 (2022.01)

(52) Cooperative Patent Classification (CPC):
**G16H 10/20; G06F 16/3329; G06F 16/3331;**
**G06F 40/30; G16H 50/20; G16H 50/30;**
G06F 40/211; G06F 40/284; G06F 40/56;
G16H 80/00

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(71) Applicant: **Fujitsu Limited**
**Kawasaki-shi, Kanagawa 211-8588 (JP)**

(72) Inventors:
• **CETINA, Kendrick**
 **28011 Madrid (ES)**

• **GARCIA SANTA, Nuria**
 **28010 Madrid (ES)**

(74) Representative: **Haseltine Lake Kempner LLP**
**Cheapside House**
**138 Cheapside**
**London EC2V 6BJ (GB)**

Remarks:
Amended claims in accordance with Rule 137(2)
EPC.

(54) **HEALTH METRIC AND DIAGNOSIS DETERMINATION**

(57)     A computer-implemented method comprising extracting information from unstructured data relating to a target patient; categorizing each of a plurality of questions as a numerical question requiring a numerical answer or a categorical question requiring a categorical answer, based on a set of question keywords and corresponding question keyword categorizations; for each question, when the extracted information includes patient information corresponding to an answer to the question, selecting that patient information as the answer, and when the extracted information does not include patient information corresponding to the answer, predicting the answer; and computing, using the selected answers and using rules, a health metric.

Figure 1

EP 4 270 403 A1

# Description

The present invention relates to a health metric and diagnosis, and in particular to a computer-implemented method, a computer program, and an information programming apparatus.

Wellbeing is an extensively studied topic that is often measured by metrics which may be focused on specific areas of interest or domains, for example social, business, health, etc. Wellbeing metrics or wellbeing Index metrics (or "health metrics") are widely used to measure wellbeing in different domains, and may be used for example to identify areas in which improvements can be made to improve a person's wellbeing and/or health.

Some examples of wellbeing index metrics are:

- Cantril's Self-Anchoring Ladder which measures present, past, and future life satisfaction with questions posed using a metaphor of a ladder.
- Gallup World Poll and Gallup Heathway's Well-being Index which measure the happiness and well-being of nations, states, and cities around the world.
- Business Well-being Metrics like the Global Reporting Initiative (GRI) and B-Corp, which provide guidelines and indicators for the areas/domains of communication and public relations, economic and financial performance, environment, labour practices and human rights, services and goods responsibility, and social performance.
- The Human Development Index (HDI) which is a single measure composed of indicators for life expectancy, mean years of education, expected years of schooling for children, and GDP per capita.

Two types of wellbeing metrics can be considered based on the way they are calculated:

1. Objective metrics - based on data that is observable such as average life expectancy, graduation rates, and income.
2. Subjective metrics - based on data gathered through surveys (also called questionnaires, polls, or assessments). Subjective wellbeing metrics measure affect (emotions), eudaimonia (flourishing), satisfaction with life, and the conditions of life (also called domains or areas).

In light of the above, a health metric extraction method is desired.

According to an embodiment of a first aspect there is disclosed herein a computer-implemented method comprising: extracting information from unstructured data relating to a target patient; categorizing each of a plurality of questions (in a questionnaire for determining a wellbeing metric) as a numerical question requiring a numerical answer or a categorical question requiring a categorical answer, based on a set (or dictionary) of question keywords and corresponding question keyword categorizations; and for each question, when/if the extracted information includes patient information corresponding to an answer to the question, selecting/storing that patient information as the answer, and when/if the extracted information does not include patient information corresponding to the answer, predicting the answer, wherein, for each question, when/if the question is a numerical question requiring a numerical answer, predicting the numerical answer comprises: extracting characteristic keywords from the question; computing a relevance measure of at least one of the characteristic keywords in a document from the unstructured data relating to the target patient (compared to all documents in the unstructured data relating to a target patient); computing a relevance measure of the at least one characteristic keyword in each of a plurality of documents (from further unstructured data) relating to other patients (compared to the plurality of documents (from the further unstructured data) relating to the other patients); identifying one of the plurality of documents relating to the other patients in which the relevance measure of the at least one characteristic keyword is most similar to the relevance measure of the at least one characteristic keyword, (respectively), in the document relating to the target patient; extracting other patient information corresponding to the numerical answer from the identified document; and selecting/storing the extracted other patient information as the numerical answer, and when/if the question is a categorical question requiring a categorical answer, predicting the categorical answer comprises: using a machine-learning neural network / model trained to predict a next word in a sequence of words from the unstructured data relating to the target patient given a plurality of words preceding the next word in the sequence, predicting a subject next word in a subject sequence of words comprising a plurality of words extracted from the question; and selecting/storing a categorical answer based on the predicted subject next word, wherein the computer-implemented method further comprises determining/computing, using the selected answers and using rules (for interpreting the answers or for computing a wellbeing metric), a health metric (or wellbeing metric).

Extracting the information from the unstructured data relating to the target patient may comprise (extracting social history information by): parsing at least one sentence in the unstructured data to generate at least one constituent sentence; searching for topic keywords in the at least one constituent sentence and categorizing the at least one constituent sentence as corresponding to at least one of a plurality of (social history) topics based on any detected topic keywords.

Extracting the information from the unstructured data relating the patient (or extracting the social history information) may comprise: detecting any number in the at least one constituent sentence; when a number is detected, using dependency parser analysis with the detected number as a root of dependency to detect a noun-chunk of text dependent on the detected number; and extracting the detected number and the detected noun-chunk as a value and a measurement unit.

Parsing the at least one sentence in the unstructured data to generate the at least one constituent sentence may comprise: detecting at least one verb in the sentence; for each at least one detected verb, (using dependency parser analysis) extracting a noun preceding the verb as an anterior argument and extracting at least one noun-chunk (directly) following the verb as at least one posterior argument to generate at least one (said) constituent sentence comprising the anterior argument followed by the verb followed by a said (or the at least one) posterior argument.

A noun-chunk may be a (base noun) phrase consisting of a noun and at least one word that describes the noun (and may be defined by a language model used in the dependency parser analysis).

Extracting the information from the unstructured data relating the patient (or extracting the social history information) may comprise, before searching for topic keywords, using a trained sentence classification model to select at least one constituent sentence comprising relevant (social history) information and discarding any other constituent sentence.

For each topic, the topic keywords may comprise words related to the topic, optionally based on a sense2vec model.

The plurality of topics may comprise any of drug, drinker, smoker, sleep, exercise, and meal.

Categorizing each of a plurality of questions may comprise, for each question, searching for the question keywords in the question and, when/if a said question keyword is identified in the question, assigning the question keyword categorization corresponding to the question keyword.

The set of question keywords and corresponding question keyword categorizations may comprise a list of question keywords and, for each question keyword, a corresponding categorization of numerical or categorical.

Categorizing each of the plurality of questions may comprise, for each question, searching for the question keywords in the question and, when/if a said question keyword is identified in the question, assigning the question the categorization corresponding to the question keyword in the set of question keywords and corresponding question keyword categorizations.

The set of question keywords and question keyword categorizations may comprise (any of) the following question keywords with the categorization of categorical: what; which; who; where; why; when; how; whose; what kind; what type; which kind; which type; and how much.

The set of question keywords and question keyword categorizations may comprise (any of) the following question keywords with the categorization of numerical: how often; how long; and how many.

Categorizing each of a plurality of questions may comprise, for each question, categorizing the question as a categorical question requiring a categorical answer when/if the question includes any of the following question keywords: what; which; who; where; why; when; how; whose; what kind; what type; which kind; which type; and how much.

Categorizing each of a plurality of questions may comprise, for each question, categorizing the question as a numerical question requiring a numerical answer when/if the question includes any of the following question keywords: how often; how long; and how many.

Categorizing each of a plurality of questions may comprise, for each question, searching for the question keywords in the question and, when/if no question keyword is identified in the question, categorizing the question as a categorical question.

The method may further comprise classifying at least one question as a fixed-set question requiring an answer from a set of possible answers in the question (or an open question requiring an open answer).

The method may further comprise classifying each categorical question as a fixed-set question requiring an answer from a set of possible answers or an open question requiring an open answer.

Classifying at least one/each question may comprise identifying the (or a) noun that appears first in the question as an initial noun, and, if an adjective-chunk directly follows the initial noun, classifying the question as a fixed-set question.

The method may further comprise selecting the adjective-chunk directly following the initial noun as the set of possible answers to the question.

An adjective-chunk may be a sequence of words appearing in the question comprising (or consisting of) a plurality of adjectives separated by (only) at least one punctuation sign and/or conjunction word.

The method may further comprise selecting the adjectives in the adjective chunk as the possible answers to the question.

The method may further comprise using a set/dictionary of nouns, a set/dictionary of adjectives, and a set/dictionary of punctuation signs and conjunction words to identify the initial noun and the adjective-chunk.

The method may further comprise using constituency analysis and/or part of speech, POS, tags, to identify the initial noun and the adjective-chunk.

The method may further comprise, for each numerical question requiring a numerical answer to be predicted: computing, for each of the characteristic keywords extracted from the question, a relevance measure of the characteristic keyword in a document from the unstructured data relating to the target patient (compared to all/a set of documents in the unstructured data relating to a target patient); computing, for each of the characteristic keywords extracted from the question, a relevance measure of the characteristic keyword in each of a plurality of documents relating to other patients (compared to the plurality of or a set of the documents relating to the other patients); identifying one of the plurality of documents relating to the other patients in which the relevance measures of the characteristic keywords are most similar to the relevance measures of the characteristic keywords, (respectively), in the document relating to the target patient.

Extracting characteristic keywords from a said numerical question may comprise cleaning the question using stemming and stop-words removal and extracting the remaining words as characteristic keywords.

Stemming may comprise removing at least one character from the end of at least one word of the question to obtain the stem/root of the word.

Stop-words removal may comprise identifying stop-words in the question using a set/dictionary of stop-words and removing the identified stop-words.

The set/dictionary of stop-words may comprise articles, prepositions, pronouns, and conjunctions.

The relevance measure (of the at least one characteristic keyword in the document from the unstructured data relating to the target patient and of the at least one characteristic keyword in each of the plurality of documents (from the further unstructured data) relating to other patients) is (or comprises) the term frequency-inverse document frequency, TF-IDF, of the characteristic keyword.

Computing the relevance measure of at least one of the characteristic keywords in the document from the unstructured data relating to the target patient may comprise, (for each at least one characteristic keyword), computing the term frequency-inverse document frequency, TF-IDF, of the characteristic keyword by: calculating a term frequency of the characteristic keyword in the document; calculating the inverse document frequency of the characteristic keyword in all documents in the unstructured data relating to the target patient; and computing the product of the term frequency and the inverse document frequency.

Calculating the inverse document frequency of the characteristic keyword in all documents in the unstructured data relating to the target patient may comprise dividing the number of documents in the unstructured data relating to the target patient by the number of those documents which include the key word to obtain an initial value, and calculating the logarithm of the initial value.

Computing the relevance measure of at least one characteristic keyword in each of a plurality of documents (from further unstructured data) relating to other patients may comprise, for each document (and for each at least one characteristic keyword), computing the term frequency-inverse document frequency, TF-IDF, of the characteristic keyword by: calculating a term frequency of the characteristic keyword in the document; calculating the inverse document frequency of the characteristic keyword in all documents (in the further unstructured data) relating to the other patients; and computing the product of the term frequency and the inverse document frequency.

Calculating the inverse document frequency of the characteristic keyword in all documents (in the further unstructured data) relating to the other patients may comprise dividing the number of documents (in the further unstructured data) relating to the other patients by the number of those documents which include the key word to obtain an initial value, and calculating the logarithm of the initial value.

Calculating the term frequency of a said characteristic keyword in a said document may comprise: counting the number of times the characteristic keyword appears in the document and optionally dividing the count by a length (in words) of the document or by the term frequency of the most frequent word in the document; or counting the number of times the characteristic keyword appears in the document and computing the logarithm of the count; or counting the number of times the characteristic keyword appears in the document, dividing the count by a length (in words) of the document or by the term frequency of the most frequent word in the document to obtain a normalized frequency, and computing the logarithm of the value (1 + the normalized frequency).

The method may further comprise, if/when relevance measures are computed for a plurality of documents in the unstructured data relating to the target patient, selecting the document which includes at least one appearance of the highest number of/most characteristic keywords from the question. Identifying one of the plurality of documents relating to the other patients may comprise identifying one of the plurality of documents relating to the other patients in which the relevance measure of the at least one characteristic keyword is most similar to the relevance measure of the at least one characteristic keyword, (respectively), in the selected document.

Identifying one of the plurality of documents relating to the other patients may comprise identifying one of the plurality of documents relating to the other patients in which the relevance measure of the at least one characteristic keyword is most similar to the relevance measure of the at least one characteristic keyword, (respectively), in the document in the unstructured data relating to the target patient.

The method may further comprise, before calculating any relevance measures, limiting the documents in the unstructured data and in the further unstructured data, for which relevance measures are to be calculated, to relevant documents by using a latent Dirichlet allocation, LDA.

Identifying one of the plurality of documents relating to the other patients in which the relevance measure of the at least one characteristic keyword is most similar to the relevance measure of the at least one characteristic keyword, (respectively), in the document in the unstructured data relating to the target patient may comprise, when the at least one characteristic keyword comprises a plurality of characteristic keywords: for each of the plurality of documents relating to the other patients and for each characteristic keyword, assigning a similarity score to the relevance measure of the characteristic keyword in the document of the plurality of documents relating to the other patients depending on how similar the relevance measure is to the relevance measure of the characteristic keyword in the document in the unstructured data relating to the target patient; for each of the plurality of documents relating to the other patients, aggregating the similarity scores for the document to obtain an overall similarity score; and identifying the document with the overall similarity score indicating the most similarity (i.e. the highest or lowest overall similarity score depending on how the similarity scores are assigned).

Assigning a similarity score to the relevance measure of the characteristic keyword in the document of the plurality of documents relating to the other patients depending on how similar the relevance measure is to the relevance measure of the characteristic keyword in the document in the unstructured data relating to the target patient may comprise: assigning a first value as the similarity score when a difference between the relevance measure of the characteristic keyword in the document of the plurality of documents relating to the other patients and the relevance measure of the characteristic keyword in the document in the unstructured data relating to the target patient is less than a threshold difference; and assigning a second value as the similarity score when a difference between the relevance measure of the characteristic keyword in the document of the plurality of documents relating to the other patients and the relevance measure of the characteristic keyword in the document in the unstructured data relating to the target patient is more than or equal to the threshold difference.

The method may further comprise, before using the neural network/model, training the neural network/model by: assembling training data by extracting sequences of words from the unstructured data and/or from the constituent sentences in the extracted information, removing the last word of each sequence to generate training sequences of words; and training the neural network/model to predict the next word in each training sequence of words, using for each training sequence of words the removed last word as ground-truth data.

The neural network/model may be a sequential model, optionally with an initial embedding layer, a Long short-term memory, LSTM, layer (with 1000 units) that is passed through another LSTM layer (with 1000 units) that is passed through a hidden layer (with 1000 node units) using a dense layer function (with a rectified linear unit, ReLU, as the activation).

The neural network/model may comprise, as a last layer, a softmax activation.

The subject sequence of words may comprise the last n words of the question, n being an integer more than 1.

If/when the question is a fixed-set question and a categorical question, selecting/storing the categorical answer based on the predicted subject next word may comprise: if/when the predicted subject next word is not one of the possible answers, finding at least one word similar to each possible answer (as at least one similar word corresponding to each possible answer), and if/when the predicted subject next word is the same as one of the at least one similar words, selecting/storing the possible answer corresponding to the predicted subject next word as the categorical answer.

If/when the question is a fixed-set question and a categorical question, selecting/storing the categorical answer based on the predicted subject next word may comprise: if/when the predicted subject next word is one of the possible answers, selecting/storing the predicted subject next word as the categorical answer.

If/when the question is an open question and a categorical question, selecting/storing the categorical answer based on the predicted subject next word may comprise: selecting/storing the predicted subject next word as the categorical answer.

Predicting the subject next word may comprise predicting a plurality of preliminary subject next words and selecting one of the preliminary subject next words as the predicted subject next word.

Predicting the subject next word may comprise predicting a plurality of preliminary subject next words and corresponding scores/probabilities. Selecting one of the preliminary subject next words as the predicted subject next word may comprise selecting the preliminary subject next word with the highest score/probability as the predicted subject next word.

If/when the question is a fixed-set question and a categorical question: selecting one of the preliminary subject next words as the predicted subject next word may comprise, if one of the preliminary subject next words is the same as a possible answer, selecting that preliminary subject next word as the predicted subject next word; and selecting/storing the categorical answer based on the predicted subject next word may comprise selecting/storing the predicted subject next word as the categorical answer.

If/when the question is a fixed-set question and a categorical question: selecting one of the preliminary subject next words as the predicted subject next word may comprise, if/when none of the preliminary subject next words is the same a possible answer, finding at least one word similar to each possible answer (as at least one similar word corresponding to each possible answer), and if/when one of the preliminary subject next words is the same as one of the at least one similar words, selecting that preliminary subject next word as the predicted subject next word; and selecting/storing the categorical answer based on the predicted subject next word may comprise selecting/storing the predicted subject next word as the categorical answer.

The health metric may be a wellbeing metric.

Computing the health metric may comprise adding a metric score to a health metric for each of the plurality of questions.

Each metric score may be weighted by a weighting corresponding to the question.

The rules may define the weightings corresponding to the questions.

The method may further comprise determining and outputting a diagnosis according to the health metric.

Determining the diagnosis may comprise determining whether or not the target patient suffers from depression.

The rules may define, for each categorical question, a metric score to be added to the health metric for each possible answer to the question.

The questions may be categorical questions requiring categorical answers. The rules may define for each question a metric score to be added to the health metric for the question for each possible answer to the question.

The rules may define a diagnosis to be output based on the health metric.

Determining the diagnosis may comprise determining whether or not the target patient suffers from alcoholism.

The questions may comprise questions relating to at least one of: how often the target patient consumes alcohol; how often the target patient consumes more than a threshold amount of alcohol (on a single occasion or in a day); how often during a time window (a year) the target patient has failed to do what was normally expected of them because of alcohol; and how often during a time window (a year) the target patient was unable to stop drinking alcohol once they started drinking alcohol (on a single occasion or in a day).

According to an embodiment of a second aspect there is disclosed herein a computer program which, when run on a computer, causes the computer to carry out a method comprising: extracting information from unstructured data relating to a target patient; categorizing each of a plurality of questions (in a questionnaire for determining a wellbeing metric) as a numerical question requiring a numerical answer or a categorical question requiring a categorical answer, based on a set (or dictionary) of question keywords and corresponding question keyword categorizations; and for each question, when/if the extracted information includes patient information corresponding to an answer to the question, selecting/storing that patient information as the answer, and when/if the extracted information does not include patient information corresponding to the answer, predicting the answer, wherein, for each question when/if the question is a numerical question requiring a numerical answer, predicting the numerical answer comprises: extracting characteristic keywords from the question; computing a relevance measure of at least one of the characteristic keywords in a document from the unstructured data relating to the target patient (compared to all documents in the unstructured data relating to a target patient); computing a relevance measure of the at least one characteristic keyword in each of a plurality of documents (from further unstructured data) relating to other patients (compared to the plurality of documents (from the further unstructured data) relating to the other patients); identifying one of the plurality of documents relating to the other patients in which the relevance measure of the at least one characteristic keyword is most similar to the relevance measure of the at least one characteristic keyword, (respectively), in the document relating to the target patient; extracting other patient information corresponding to the numerical answer from the identified document; and selecting/storing the extracted other patient information as the numerical answer, and when/if the question is a categorical question requiring a categorical answer, predicting the categorical answer comprises: using a machine-learning neural network / model trained to predict a next word in a sequence of words from the unstructured data relating to the target patient given a plurality of words preceding the next word in the sequence, predicting a subject next word in a subject sequence of words comprising a plurality of words extracted from the question; and selecting/storing a categorical answer based on the predicted subject next word, wherein the method further comprises determining/computing, using the selected answers and using rules (for interpreting the answers or for computing a wellbeing metric), a health metric (or wellbeing metric).

According to an embodiment of a second aspect there is disclosed herein an information processing apparatus comprising a memory and a processor connected to the memory, wherein the processor is configured to: extract information from unstructured data relating to a target patient; categorize each of a plurality of questions (in a questionnaire for determining a wellbeing metric) as a numerical question requiring a numerical answer or a categorical question requiring a categorical answer, based on a set (or dictionary) of question keywords and corresponding question keyword categorizations; and for each question, when/if the extracted information includes patient information corresponding to an answer to the question, select/store that patient information as the answer, and when/if the extracted information does not include patient information corresponding to the answer, predict the answer, wherein, for each question when/if the question is a numerical question requiring a numerical answer, predicting the numerical answer comprises: extracting characteristic keywords from the question; computing a relevance measure of at least one of the characteristic keywords in a document from the unstructured data relating to the target patient (compared to all documents in the unstructured data relating to a target patient); computing a relevance measure of the at least one characteristic keyword in each of a plurality of documents (from further unstructured data) relating to other patients (compared to the plurality of documents (from the further unstructured data) relating to the other patients); identifying one of the plurality of documents relating to the other patients in which the relevance measure of the at least one characteristic keyword is most similar to the relevance measure of the at least one characteristic keyword, (respectively), in the document relating to the target patient; extracting other patient information corresponding to the numerical answer from the identified document; and selecting/storing the extracted other patient information as the numerical answer, and when/if the question is a categorical question requiring a categorical answer, predicting the categorical answer comprises: using a machine-learning neural network / model trained to predict a next word in a sequence of words from the unstructured data relating to the target patient given a plurality of words preceding the next word in the sequence, predicting a subject next word in a subject sequence of words comprising a plurality of words extracted from the question; and selecting/storing a categorical answer based on the predicted subject next word, wherein the processor is further configured to determine/compute, using the selected answers and using rules (for interpreting the answers or for computing a wellbeing metric), a health metric (or wellbeing metric).

Features relating to any aspect/embodiment may be applied to any other aspect/embodiment.

Reference will now be made, by way of example, to the accompanying drawings, in which:

Figure 1 is a diagram illustrating a method;
Figure 2 is a diagram useful for understanding embodiments;
Figure 3 is a diagram of a wellbeing metric representation;
Figure 4 is a diagram useful for understanding embodiments;
Figure 5 is a diagram illustrating a method;
Figure 6 is a diagram illustrating a method;
Figure 7 is a diagram illustrating a method;
Figure 8 is a diagram illustrating a neural network configuration;
Figure 9 is a diagram illustrating a table;
Figure 10 is a diagram illustrating a table;
Figure 11 is a diagram illustrating a table;
Figure 12 is a diagram illustrating tables;
Figure 13 is a diagram illustrating a table;
Figure 14 is a diagram illustrating a table and a wellbeing metric representation;
Figure 15 is a diagram illustrating a table;
Figure 16 is a diagram illustrating tables;
Figure 17 is a diagram illustrating tables;
Figure 18 is a diagram illustrating a table; and
Figure 19 is a diagram illustrating an apparatus;

The following definitions may be used in the description but are not exhaustive.

- *Text Mining:* the process of deriving high-quality information from text. The process or practice of examining large collections of written resources in order to generate new information. The goal of text mining is to discover relevant information in text by transforming the text into data that can be used for further analysis.

- *Natural language processing (or NLP)*: a step in Text Mining involving a linguistic analysis that helps a machine "read" text. NLP uses a variety of methodologies to decipher ambiguities in human language. NLP is a field of computer science, artificial intelligence, and computational linguistics concerned with the interactions between computers and human (natural) languages.

- *Machine Learning (ML):* the subfield of computer science that may be considered, broadly speaking, to give computers the ability to learn without being explicitly programmed. ML involves the study and construction of algorithms that can learn from and make predictions on data.

- *Deep Learning (DL)*: a part of a broader family of machine learning methods based on learning data representations, as opposed to task-specific algorithms.

- *Artificial Neural Network/Neural Network (ANN/NN):* an information processing paradigm that is inspired by the way a biological nervous system, such as the brain, processes information. ANN/NNs may be used to extract patterns and detect trends that are too complex to be noticed by either humans or other computer techniques.

- *Named Entity Recognition (NER)*: a subtask of information extraction that seeks to locate and classify named entities mentioned in unstructured text into categories.

- *Relation Extraction*: the task of extracting semantic relationships from text. Extracted relationships usually occur between two or more entities of a certain type (e.g. Person, Organisation, Location) and fall into a number of semantic categories (e.g. married to, employed by, lives in).

- *Wellbeing index metric or wellbeing metric or health metric:* may be considered a measure of health, either overall or in a specific area (i.e. a depression metric or an alcoholism metric). A health/wellbeing metric may be used to determine a diagnosis, e.g. of depression or alcoholism. A health/wellbeing metric may also be considered a measure of overall wellbeing and progress. Assessing such progress may involve looking not at the functioning of the economic system and at the diverse experiences and living conditions of people.

- *Social History:* In medicine, a social history (abbreviated "SocHx") may be considered a portion of the medical history (and thus a portion of e.g. an admission note) addressing familial, occupational, and recreational aspects of a patient's personal life (these aspects may have the potential to be clinically significant).

- *Family History:* In medicine, a family history (FH or FHx) may include/consist of information about disorders from which direct blood relatives of the patient have suffered. Genealogy may include little of the medical history of the family of a patient, but medical history may be considered a specific subset of the total history of a family. Accurate knowledge of a patient's family history may help in identification of a predisposition to developing certain illnesses/diseases/conditions, which may inform clinical decisions and allow effective management or even prevention of illnesses/diseases/conditions.

Embodiments disclosed herein may automatically generate a Subjective Wellbeing Index Metric using Natural Language Processing (NLP) and Machine Learning (ML) technologies and using unstructured text data.

Embodiments may comprise or utilize a tool that uses collected data from different sources and automatically computes standardized subjective wellbeing index metrics.

Embodiments may include the following technical features:

- Automatic generation of a (subjective) wellbeing index metric by automatic extraction of relevant data (e.g. Social history and/or Family history information) included in unstructured documents through NLP and ML operations.
- Use of ML and NLP techniques to generate responses to surveys and questionnaires for health metric calculation.
- Automatic generation of missing data by using natural language Question-Answering ML models and e.g. Social History information, when there is missing information in the unstructured data relating to a target patient.
- Module to automatically generate a wellbeing index metric from raw text using computation rules of Wellbeing Index Metric questionnaires, for example.
- NLP module for Social History extraction and classification of extracted information into categories (smoke, drinks, drugs, etc.) with numerical values.
- A Numerical Answer prediction model and a Categorical Answer prediction model for generating answers to questions from raw text data.

  - Numerical Answer prediction model: Uses keyword extraction and TF-IDF (term frequency-inverse document frequency) operations over data to find similar documents and predicts a numerical answer using a similar document.

- Categorical Answer prediction model: Generates a predicted categorical answer using a Next-Word prediction model.

Embodiments may be integrated into other systems, e.g. a system for Visual Diagrams generation. Embodiments may include/achieve the following technical features/advantages:

- Automatic index metric generation: A (subjective) wellbeing index metric is calculated by a system automatically by a combination of prediction models to extract and estimate values/answers required to compute the wellbeing index metric. That is, user interaction (e.g. manually answering questionnaires) is not required to compute the index metric.
- Social & Family History: The system may use NLP models and operations specific to the information extraction of Social and Family history from raw unstructured text data.
- Self-adapting wellbeing index metric: The system may use learning models and operations to answer specific questions e.g. in a questionnaire, by an automatic process that adapts an answer to a specific question.

Some existing methodologies for determining a wellbeing index may have any of the following drawbacks:

- User input is actively required (therefore input devices, sensors, and text forms, among other apparatuses/elements, are required). Waiting for a user to answer questions through user input is time consuming.
- No ability to take advantage of (i.e. utilize) data already acquired for reasons outside of wellbeing (Electronic Health Records, other feedbacks, work logs, etc.)
- No ability to predict answers to questions. This may be important because wellbeing metric computation may be sensitive to missing information. In some instances, there may be data points that must be present for the metric to be valid. That is, in some instances, if a questionnaire is not fully answered, the metric cannot be computed.

- Conventional index metric computation methodologies require a specific questionnaire for each metric - i.e. they are based on specific questionnaires or vice versa, and those methodologies would not work if the questions were changed. Embodiments disclosed herein are not specific to any questionnaire or set of questionnaires, i.e. they are independent of whichever questionnaire is used.

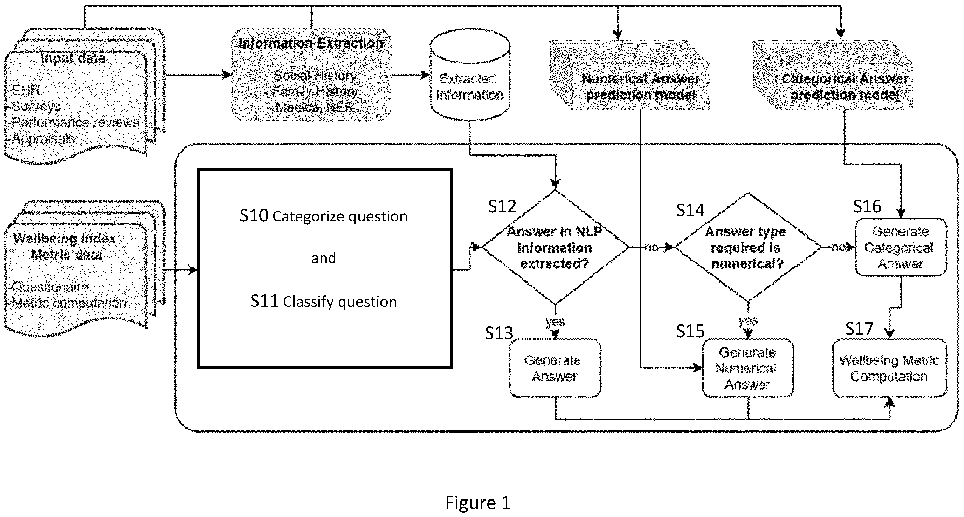

Figure 1 illustrates a method according to a running example. The method may be considered implemented by a system or an apparatus comprising modules/units in some instances. The , method receives two inputs:

- Input data: Text data related to a person/patient/user, referred to as a target patient. This data may be any type of text data related to the wellbeing of the target patient (e.g. Electronic Health Records (EHR), surveys, performance reviews, personal information, etc.). The input data may also include text data related to other patients. The text data is unstructured data.

- Wellbeing Index Metric data or wellbeing/health metric data: Elements required to compute a wellbeing metric: questions (the answers to which to be used in computing the health metric) and rules for computing the health metric (for example, each answer may correspond to a value and be assigned a value-weight (e.g. by topic: physical, social, emotional, etc.), and the summation of the values according to the value-weights may give the health metric).

The input data may be considered input to three modules:

- A module for information extraction which receives the input data and performs three processes (described in more detail below) to extract relevant information:

    1. Social history extraction
    2. Family history extraction
    3. Medical Named Entity Recognition (NER) extraction

- A module to implement a numerical answer prediction model, which receives the input data along with external publicly available medical data to generate answers to questions requiring a numerical-type answer, e.g., "How many days per week do you exercise?"

- A module to implement a categorical answer prediction model, which receives the input data along with pre-trained models (e.g. sense2vec) to generate answers to questions requiring a categorical-type answer, e.g., "Do you smoke?"

An apparatus/system implementing the method of Figure 1 may comprise an Answer Manager (not shown) for analysing the questions in the wellbeing index metric data and categorizing each question. The answer manager may be considered to carry out steps S10 to S17. The operations of the Answer Manager may be considered to comprise two overall functions:

    1. Question categorization: For each question the Answer Manager determines:

    ■ The answer-type: Depending on the question the answer to the question may be:

    • Numerical

    • Categorical

    ■ The answer-value-type: Depending on whether a set of possible answers is included in by the question, the possible answers can be:

    • Open: if the answer can take any numerical/categorical value. E.g., with *"how many?" "how much?"* type questions.
    • Fixed-set: If the question provides a finite set of possible answers. E.g., Given the question: *"are you satisfied or dissatisfied with your work?"* the fixed set of answers is [*'satisfied', 'dissatisfied'*]

    2. Answer generation: This function uses the question categorization information and the three modules (Information Extraction, Numerical & Categorical Answer prediction models) to generate an answer to each question.

The apparatus/system implementing the method of Figure 1 (e.g. the answer manager, specifically) computes a wellbeing index metric according to the procedures in the wellbeing index metric data.
The method in Figure 1 is described in more detail below.
The answer manager which implements the method of Figure 1 in a running example receives the inputs:

- Wellbeing index metric data
- Output from the module for information extraction
- Output from the module for the numerical answer prediction model
- Output from the module for the categorical answer prediction model;

and generates as an output:

- A Wellbeing Index Metric.

Step S10 comprises categorizing each of a plurality of questions (in a questionnaire for determining a wellbeing metric) as a numerical question requiring a numerical answer or a categorical question requiring a categorical answer, based on a set or dictionary of question keywords and corresponding question keyword categorizations.
That is, in a running example, in step S10 the answer manager categorizes all questions in a Wellbeing Index Metric data questionnaire. These questions can be part of one or more standardized questionnaires e.g. for computing different health/wellbeing indexes.

Step S10 utilizes a mapping of the most popular question-words or question keywords and corresponding categorizations. I.e. a set or dictionary of question keywords and corresponding question keyword categorizations is utilized and then each question may be processed in the following way:

When the stemmed form of a question-word is present in the question,
then assign the corresponding categorization.
If the question does not include any of the question-words,
then categorize the question as categorical.

The following is a list of question keywords or question-words that, if detected in a question would lead to the question being categorized as a categorical question requiring a categorical answer:

What (for a thing, when there are many things)
Which (for a thing, when there aren't many things)
Who (for a person)
Where (for a place)
Why (for a reason)
When (for a time)
How (for a method)
Whose (to ask about possession)
What kind / What type... ?
Which kind / which type... ?
How much...? (to talk about quantity in uncountable nouns)

The following is a list of question keywords or question-words that, if detected in a question would lead to the question being categorized as a numerical question requiring a numerical answer:

How often...? (to talk about frequency)
How long... ? (to talk about duration)
How many...? (to talk about quantity in countable nouns)

Step S10 may be considered a categorization of each question or each required answer, or both.

Step S11 comprises classifying each (or at least one) question as a fixed-set question requiring an answer from a set of possible answers in the question or an open question requiring an open answer.

Step S11 may be referred to (as above, previously) as a second categorization or answer-value type categorization whilst step S10 may be referred to (as above, previously) as a first categorization or answer-type categorization.

In a running example, for each question in the wellbeing questionnaire, step S11 is carried out for a question as follows:

If there is no fixed set of possible answers in the question,
then the question is categorized as open.
If there is a fixed set of possible answers in the question,
then the question is categorized as fixed-set
and the set of possible answers is extracted and associated with the question to be used in an Answer Generation Flow.

The answer generation flow is a combination of steps of the method for generating an answer to a question, described later below.

In order to detect a fixed set of possible answers in a question, constituency parsing analysis is performed over the question. Constituency parsing analysis uses Part-of-Speech tags (POS-Tags) obtained with open libraries like Spacy (https://spacy.io/usage/linguistic-features). For the following analysis only the Adjectives and Nouns from the Part-of-Speech tags in the question are considered.

The list of POS-Tags used for Answer-value-type categorization (step S11) is:

JJ: adjective; e.g. big
JJR: adjective, comparative; e.g. bigger
JJS: adjective, superlative; e.g. biggest
NN: noun, singular or mass; e.g. door
NNS: noun, plural; e.g. doors
NNP: proper noun, singular: e.g. John
NNPS: proper noun, plural; e.g. Vikings

The fixed set of possible answers, if present in the question, belongs to an adjective chunk closest to an initial noun in the question. An adjective-chunk is a group of words with the POS-tag starting with 'J' and separated only by punctuation signs or conjunction words (POS-tag 'CC'). That is, an adjective-chunk is a sequence of words appearing in the question consisting of a plurality of adjectives separated by only at least one punctuation sign and/or conjunction word. The initial noun is the first noun appearing in the sentence.

To obtain the set of possible answers the following algorithm may be used:
For all adjective-chunks in the sentence:
If the adjective-chunk is the closest to a noun (POS-Tag that starts with 'N') Then save adjective-chunk

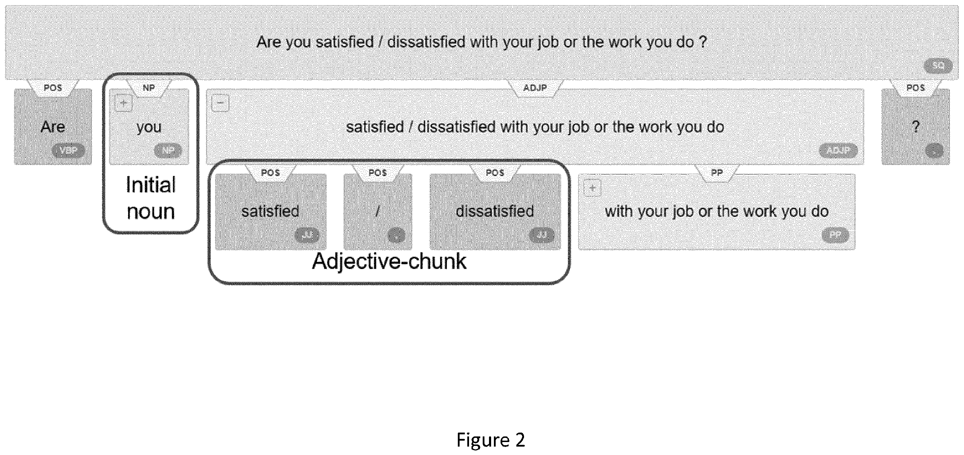

To exemplify this algorithm, consider the question "Are you satisfied / dissatisfied with the work you do?". Figure 2 illustrates the question with its corresponding POS-tags.

Following the algorithm, the fixed set of possible answers ['satisfied' 'dissatisfied'] is obtained.

In other words, step S11 comprises, for a said question, identifying the noun that appears first in the question as an initial noun, and, if an adjective-chunk directly follows the initial noun, classifying the question as a fixed-set question. Step S11 further comprises selecting the adjective-chunk directly following the initial noun as the set of possible answers to the question, i.e. selecting the adjectives in the adjective chunk as the possible answers to the question. The initial noun and adjective-chunk(s) are detected using POS-tagging.

Answer manager: Answer Generation Flow

The Answer Generation Flow comprises steps S12 to S16 and is used to followed an answer for each question. The answer generation flow may be summarized as follows:

> For each question in the wellbeing questionnaire:
> If the question requires an answer-type Categorical Then use Answer-value-type information (open or fixed set of answers), Module: Categorical Answer prediction model and Extracted information from Module: Information Extraction to generate an answer.
> If the question requires an answer-type Numerical Then use Numerical Answer prediction model and Extracted information from Module: Information Extraction to generate an answer.

The answer generation flow for a question may be considered as follows. Step S12 comprises determining whether the answer to a question can be found in information extracted from the input data. If "yes", the method proceeds to step S13 which comprises generating an answer based on the extracted information. If "no" after step S12, the method proceeds to step S14 which comprises determining if the question is a numerical question requiring a numerical answer. In step S14, the result of step S10 may be referred to. If "yes" at step S14, the method proceeds to step S15 which comprises generating a numerical answer using the numerical answer prediction model. If "no" at step S14, the method proceeds to step S16 which comprises generating a categorical answer using the categorical answer prediction model. The method proceeds (from step S13 or S15 or S16, depending on which step was implemented) to step S17 which comprises computing a health/wellbeing metric.

Steps S10 to S16 are carried out for a plurality of questions, e.g. each question in a questionnaire for computing a health/wellbeing metric.

Answer manager: Subjective wellbeing index metric computation (Step S17)

With a plurality of questions (e.g. all questions in the wellbeing questionnaire) having been answered, the Wellbeing index metric data is used to assign a numerical value to each answer and step S17 may further comprise summing the values according to a weight of each question prescribed by the wellbeing index metric data.

In a particular example, the wellbeing questionnaire relates to a plurality of domains/areas, which may be referred to as wellbeing dimensions. The wellbeing dimensions considered depend on the health/wellbeing metric being computed, e.g. on the Wellbeing index metric data. They may comprise any of the following, among others:

- Emotional
- Financial
- Social
- Spiritual
- Occupational
- Physical
- Intellectual
- Environmental
- Health (including metrics that may be used to determine a diagnosis)

In an example, each question in the wellbeing questionnaire may be concerned with one of these dimensions.

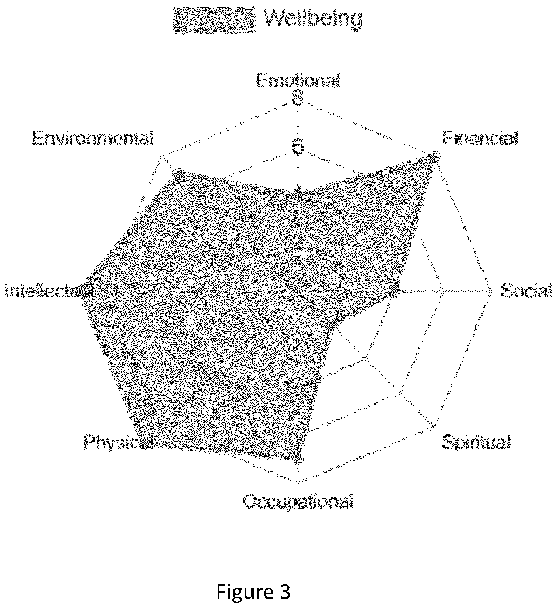

A wellbeing index may be represented with a radar chart as shown in Figure 3.

The method steps in Figure 1 may be carried out in a different order and some steps may be omitted. Each step has been described according to a particular running example. The scope of the invention is defined in the claims.

Information extraction based on the input data will now be described.

For each document in the Input Data three different types of information extractions may be performed.

Extracted Information (shown in Figure 1) may comprise a database storing entities and relationships found in the input data, associated with the relevant document. The three types of information extraction are social history extraction, family history extraction, and medical named entity recognition (NER).

Social history extraction

Social history information is extracted according to social history topics including:

> a. Exercise
> b. Drugs
> c. Smoking
> d. Meals/Nutrition
> e. Drinking
> f. Sleep

The information extraction involves the capture/extraction of any value related to each Social History topic. For example, in a given sentence from a document of the input data, "Patient smokes 20 cigarettes a day" the value "20" and the unit of measurement "a day" is associated with (or stored in association with) the entity "smokes".

Extract social history information may be considered to follow four steps:

- Special sentence split (or sentence parsing) to-kenize sentences in a more modular manner instead of a punctuation tokenization of sentences.
- Sentence classification to discard sentences that do not include social history information.
- Specialized detection of entities to categorize sentences as corresponding to the specific topics of social history.
- Regular expression to find numerical values and units of measurement.

Special sentence split (sentence parsing)

The special sentence split step comprises, for a given sentence in the input data, parsing the sentence to generate at least one constituent sentence. The special sentence split includes extracting a list of propositions, each composed of a single predicate and an arbitrary number of arguments. These extractions break syntactically complex sentences into the relationships they express, which can then be used for various downstream tasks. This is done by analysis of POS-tags and dependency parsers of language models, and may be summarized by the following:

For each verb in the sentence:
If the verb has anterior and posterior arguments then save as a constituent sentence: anterior argument + verb + posterior argument

The dependency parser analysis may make use of POS tags, e.g. to identify each verb.
As an example, consider the sentence: "Patients comes to consult complaining about chest pain, smokes 20 cigarettes per day and has a balanced diet."
Anterior and posterior arguments are found by dependency parsing based on each verb in the sentence. Each argument is grouped by noun-chunks. Dependency parsing may comprise using a dependency parser which is a trained model capable of analyzing the grammatical structure of a sentence based on the dependencies between the words in the sentence. Noun chunks are "base noun phrases" - flat phrases that have a noun as their "head". Noun chunks may be considered a noun plus the words describing the noun - for example, "the lavish green grass". Each argument found by the dependency parser and shown below comprises at least one noun chunk. The arguments (ARGs) shown below are dependent on the Verbs (V) in the sentence. This step in the algorithm looks for noun chunks before and after the verb.
The aim of this step may be considered to be to break a sentence into the smallest possible sentences each having a Subject, an action and an object. This process is optimized by finding noun chunks before and after a Verb. For each Verb there is an extraction. Each extraction finds noun chunks dependent on the VERB (using the dependency parser). Some noun chunks can be less accurate if the dependency parser model fails to relate chunks to the VERB (like the first extraction below for the verb: comes). But the optimization of the process leads to successful extractions in other cases, for example the extractions for verbs: complaining, smokes and has.
The sentence parsing (or special sentence split) may be considered to comprise detecting at least one verb in the sentence and, for each at least one detected verb, using dependency parser analysis, extracting a noun preceding the verb as an anterior argument and extracting a noun-chunk directly following the verb as a posterior argument to generate a constituent sentence comprising the anterior argument followed by the verb followed by the posterior argument.
For example, considering the above example sentence, the following extractions may be made based on the above steps:

Extraction for comes:
(ARGO: Patient) (V: comes) (ARG2: to consult complaining about chest pain, smokes) (ARG1: 20 cigarettes per day and has a balanced diet).

Extractions for consult (none):
(ARGO: Patient) comes to (V: consult) complaining about chest pain, smokes 20 cigarettes per day and has a balanced diet.

Extractions for complaining:
(ARGO: Patient) comes to consult (V: complaining) (ARG1: about chest pain), smokes 20 cigarettes per day and has a balanced diet.

Extractions for smokes:
(ARGO: Patient) comes to consult complaining about chest pain, (V:smokes) (ARG1: 20 cigarettes) (ARGM-TMP: per day) and has a balanced diet.

Extractions for has:
(ARGO: Patient) comes to consult complaining about check pain, smokes 20 cigarettes per day and (V: has) (ARG1: a balanced diet).

In the above, ARG0 indicates an anterior argument, ARG1 indicates a posterior argument, ARG2 indicates another posterior argument, V indicates a verb, and ARGM-TMP indicates a temporal argument. The temporal argument "per day" is considered in this example part of the noun chunk "20 cigarettes per day". In some implementations, temporal arguments may not be specifically extracted/noted and in that case the posterior argument ARG1 for the verb "smokes" would be "20 cigarettes per day".
Then, for each verb, if an anterior and a posterior argument are detected, they are saved as a constituent sentence with the form:

Anterior + verb + posterior. Therefore the following constituent sentences are extracted from the example sentence:

- Patient comes to consult complaining about chest pain, smokes
- Patient complaining about chest pain
- Patient smokes 20 cigarettes per day
- Patient has a balanced diet

The extractions for "comes" included two posterior arguments. The language model used in this example detected two main noun chunks ("chest pain" and "20 cigarettes") related to the verb "comes" by the dependency parser analysis. The limits and the words included in the noun-chunk are outputs of a model that in the above example is not wholly accurate. However in the above example, if two or more posterior arguments are identified, only the first posterior argument (noun chunk), i.e. the posterior argument closest to the verb, is extracted to form a constituent sentence with the verb and the anterior argument, and the second (or any further) posterior arguments are ignored. That is, only a noun chunk directly following the verb is extracted.

In the above example there are no extractions for the verb "consult". The noun-chunk "chest pain" is the closest noun chunk to the verb consult but is not determined by dependency parser analysis to be dependent on the verb "consult".

Sentence classification

The constituent sentences are classified as comprising social history information or not comprising social history information. In an example the constituent sentences are tagged as 1 or 0 if they contain social history information or not, respectively. A pre-trained language model employing Bidirectional Encoder Representations from Transformers (BERT) may be used in this step, i.e. to detect the presence of social history information. A BERT model may be fine-tuned using some extracted constituent sentences. This trained sentence classification model is used to select only constituent sentences that include social history information. A supervised learning approach may be used to train the model, in which the model receives sentences each with a label of: "containing social history information" or "not containing social history information". The sentence is transformed to the BERT format (embeddings, i.e. a numerical array/vector describing each word). The resulting trained model recognizes elements in the sentence that can be categorized as "containing social history", but it does not follow a ruled-based approach for example looking for particular words which indicate social history information. The embeddings containing the representation of words (i.e. context and meaning) are responsible for the classification.

Specialized detection of entities

After the constituent sentences have been classified, those that include social history information are categorized by topics in social history. That is, a constituent sentence is categorized as corresponding to a particular social history topic depending on what category of social history information the constituent sentence includes.

For example:

If stemming(s) of "Drugs" present in constituent sentence
then categorize constituent sentence as DRUG

If stemming(s) of "Drinking" present in constituent sentence
then categorize constituent sentence as DRINKER

If stemming(s) of "Smoking" present in constituent sentence
then categorize constituent sentence as SMOKER

If stemming(s) of "Sleep" present in constituent sentence
then categorize constituent sentence as SLEEP

If stemming(s) of "Exercise" present in constituent sentence or physical activity NER returns value in constituent sentence
then categorize constituent sentence as EXERCISE

If stemming(s) of "Meals/Nutrition" present in constituent sentence or "food" NER returns value in constituent sentence
then categorize constituent sentence as MEAL

Although "stemming" is used above ("stemming" is described later below), to categorize a constituent sentence any words/terms related to the topic may be searched for in the constituent sentence. For example, the "stemming" of topics may be obtained by stemming of concepts related to the entity/topic. For example, terms related to the topic may be obtained using a model for finding related terms and/or words/terms with similar or related meanings, e.g. the sense2vec model (https://spacy.io/universe/project/sense2vec). For instance, terms related to DRUGS obtained with the sense2vec model include: [hard drugs, illegal drugs, other drugs, hardcore drugs, drug, heroin, illicit drugs, harder drugs, prescription drugs, legal drugs, recreational drugs, more drugs, prescription pills, narcotics, meth, street drugs, illicit substances, said drugs, dangerous drugs, certain drugs, real drugs, heavy drugs, addicts, cocaine, drugs-, addictive drugs, various drugs, marijuana, drug use, other drug, Heroin, illegal drug, illegal substances].

The presence in a constituent sentence of these terms (and other terms related to other topics) is determined by pattern matching after stemming.

For some topics, NER may also be used to detect a particular topic in a constituent sentence. That is, a pre-trained NER model may be used to detect entities in a constituent sentence related to a particular topic. Many NERs, e.g. Physical activity NERs and food NERs, are publicly available in most language models.

A constituent sentence may be categorized as corresponding to more than one topic.

Regular expression for numerical values

A regular expression is a pattern describing a certain amount of text, and may be abbreviated to "regex" or "regexp". Regular expressions are used to search through data for a text string that matches the pattern of the regular expression.

In a running example, to find numbers in the text of the input data, the regular expression "[^0-9,.]" is used to find all types of numbers in the text. Dependency parser analysis is then carried out, using the found number as a root of dependency, to find an immediate noun-chunk dependent on the number. In this way a value and its measurement unit may be identified in (and extracted from) a text in the input data. In some implementations the regular expression is used on the constituent sentences and not on the raw text.

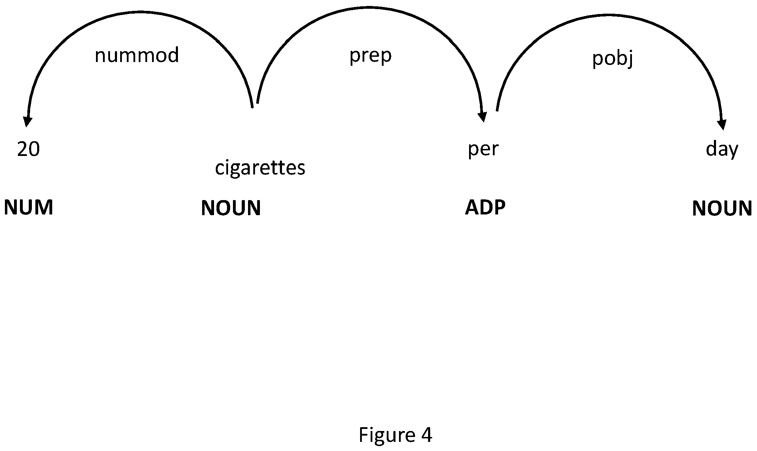

As an example, the sentence: "20 cigarettes per day" may be considered. The number 20 is captured/identified/found by the regular expression and the dependent noun-chunk is "cigarettes per day", found/identified by the dependency parser. This is illustrated in Figure 4, in which NUM indicates a number, NOUN indicates a noun, ADP indicates adposition, nummod indicates a numeric modifier, prep indicates a prepositional modifier, and pobj indicates an object of preposition. NUM, NOUN, ADP, etc., are tags which may be used by language models to identify elements in a sentence. In some implementations, the tags used are part of a standard tagging convention/system found at https://universaldependencies.org/u/dep/. The language model used in a running example is from the Spacy library, and the tags and explanations may be found at https://github.com/explosion/spaCy/blob/master/spacy/glossary.py.

Family history information extraction

Family history information is extracted from the input data (which may be referred to when describing the family history information extraction as unstructured data or unstructured (medical history) data. The input data may relate to the target patient and also to their family members. The family history information extraction may be considered to be performed by modules/engines as described below, but the operations and method steps performed by these modules/engines may be considered method steps implementable by a computer or system or apparatus (not necessarily divided into modules/engines), the same or different to that implementing the other method steps disclosed herein.

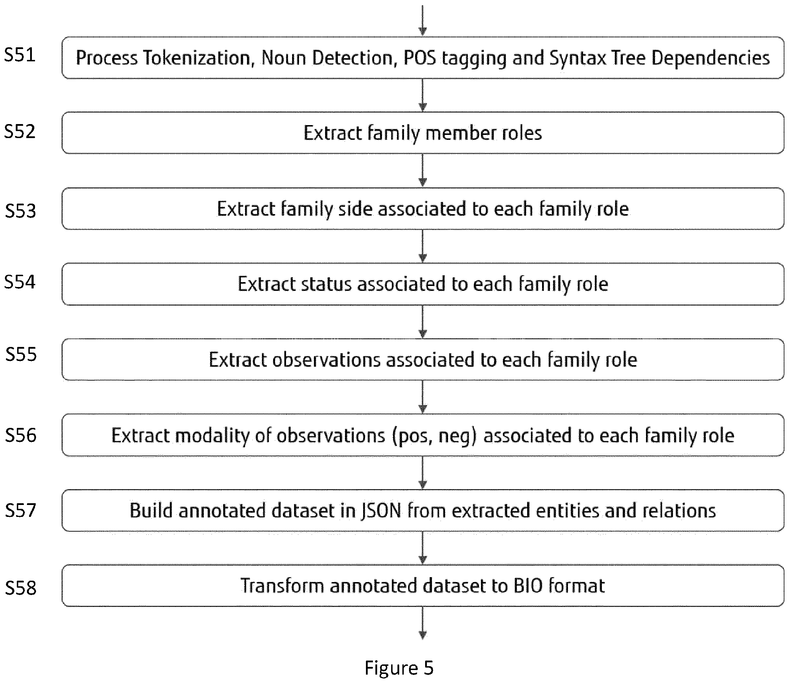

Figure 5 illustrates a method according to a running example which may be executed by a label annotator engine. The label annotator engine receives as an input text fragments of training clinical documents with Family Medical information included (unstructured training data). The label annotator engine implements a rule-based algorithm to extract family medical information from unstructured texts and create annotated datasets to be used in training a family medical history model (FMH) model. The rule-based algorithm is illustrated in the method of Figure 5.

Step S51

Step S51 comprises receiving unstructured training (medical history) data; dividing the unstructured training (medical history) data into sentences and tokenizing each sentence to extract tokens, the tokens comprising family member role keywords and/or status keywords; and performing noun detection among the tokens (and part of speech (PoS) extraction on the sentences). Step S51 may also comprise generating syntax tree dependencies for each sentence to analyse relationships between detected nouns/entities.

That is, step S51 comprises Process Tokenization, Noun Detection, POS tagging and Syntax Tree Dependencies. As the unstructured training (medical history) data, Family History information in discharge summaries is collected from public repositories. The information is filtered by section names of the document(s) to get the appropriate data. Cleaning and text processing tasks may be carried out on the collected information/data. The training data may relate to a target patient. The target patient may be the same as or different from a target patient to which the input data (not necessarily for training) relates. The training data may relate to a plurality of different families and therefore different target patients.

For the tokenization, third-party libraries may be used to divide the Family History texts into sentences and tokenize each sentence. Tokenization is a way of separating a piece of text into smaller units called tokens. Here, tokens can be either words, characters, or subwords. Noun detection and Part of Speech (POS) extraction may be carried out over the sentences to get relevant data.

Syntax tree dependencies of each sentence will be generated to analyse relationships between entities. For the next steps of the method, information processed/extracted in step S51 will aid in the extraction of entities and relations about Family Medical History.

For instance, for the sentence, "Mother and grandmother died of diabetes.", the information extracted by tokenization is, following the notation format of token --> syntax tree tag --> POS tag:

Mother--> nsubj --> NOUN
and --> cc --> CCONJ
grandmother--> conj --> NOUN
died --> ROOT --> VERB
of --> prep --> ADP
diabetes --> pobj --> NOUN
. --> punct --> PUNCT

The meanings of the POS tags are as follows: NOUN indicates a noun, VERB indicates a verb, ADP indicates an adposition, PUNCT indicates punctuation, and CCONJ indicates a coordinating conjunction. Further POS tags that may be used may be found at https://universaldependencies.org/u/pos/all.html.

The meanings of the syntax tree tags (grammatical tags of the syntax tree dependencies) are as follows: nsubj=subject (noun), ROOT=main verb, prep=preposition, punct=punctuation, cc=coordination, pobj=object of preposition. Further syntax tree tags that may be used may be found at https://downloads.cs.stanford.edu/nlp/software/dependencies_manual.pdf.

Step S51 may be considered to comprise tokenisation optionally followed by noun detection, optionally followed by POS analysis and/or syntax tree tag analysis. Noun detection may be considered to comprise using the POS tags and/or syntax tree tags. Nouns may be detected by filtering tokens to find tokens comprising an element with the POS tag NOUN.

Step S52

Step S52 comprises identifying family member role keywords among the detected nouns by comparing at least some of the detected nouns with a dictionary of family member role keywords.

That is, step S52 comprises extracting family member roles. An initial dictionary may be defined with 31 basic types (keywords) for family roles. Family roles include entities such as Father, Mother, etc. From such initial set, synsets (i.e. a set of one or more synonyms that share a common meaning) may be searched e.g. in the WordNet (http://compling.hss.ntu.edu.sg/omw/) resource (with multilingual capabilities), to construct an extended dictionary of family roles (keywords) for the desired language (e.g. English). Using this particular method, 25 extended entities are obtained, making a total number of 56 family role types (keywords) in the dictionary.

In order to return a list of family roles (keywords) from the Family History texts the nouns detected in the sentences in step S52 may be filtered using several parameters of those nouns, and then the (filtered) nouns are compared with the dictionary to match potential family roles. The resultant entities matched are outputted to the next step.

Step S53

Step S53 comprises extracting a family side associated with each family role. The keywords 'maternal' or 'paternal' are searched for. Such keywords are extracted in the tokenisation together with the associated family role keyword as a (unique) token. Therefore, it is checked whether those keywords (maternal or paternal) are present in each (noun) token including a family role (keyword). If so, the family side keyword will be associated with the specific family role keyword in a list. The complete processed list is outputted to the next step.

Step S54

Step S54 comprises, for any token comprising a family member role keyword and not a status keyword, the family member role keyword being a first family member role keyword, implementing a first status search comprising: analyzing tokens occurring in the unstructured training (medical history) data between the appearance of the first family member role keyword and the next appearance of a family member role keyword to identify a status keyword among those tokens, wherein the group including the first family member role keyword includes the identified status keyword.

Step S54 further comprises, for any token comprising a family member role keyword and not a status keyword which has not been extracted as a group comprising a status keyword after the first status search, implementing a second status search comprising: identifying a second family member role keyword occurring in the unstructured training (medical history) data before the appearance of the first family member role keyword and separated from the first family member role keyword by a conjunctive element (e.g. being a comma or the word "and"), wherein the group comprising the second family member role keyword includes the status keyword which is also included in the group comprising the first family member role keyword of the first status search.

That is, step S54 comprises extracting a status associated with each family role (keyword). A dictionary may be defined with root terms of a status such as died, alive, healthy, live, etc. Such terms may be lemmatized in order to cover all potential verbal forms or variations appearing in the texts. If the status keyword is extracted together with the family role keyword as a (unique) token, the approach in the family side extraction is followed (that is, the status keyword in the token is associated with the family member role keyword). In some implementations, only words with the POS tag of VERB or ADJ (adjective) may be compared with the dictionary of status keywords to identify status keywords.

However, there are cases where this situation does not happen. For such cases, an iterative algorithm capable of analysing text information between appearances of family roles (keywords) in the text may be implemented. This is iterated over all the tokens of the text, and detects two continuous appearances of family roles (keywords). The tokens between such appearances are compared with the dictionary and optionally lemmatizations, looking for status keywords matches. If there is match, the status (keyword) found is associated with the first family role keyword appearing before the status keyword. This part of the iterative algorithm may be referred to as a first status search.

After the first extraction of statuses for the family roles of the list over the whole text (the first status search), another part of the iterative algorithm may be implemented to identify missing statuses due to conjunctive phrases. This may be referred to as a second status search and may be considered a second iteration of the status search. For instance, for the sentence *'His mother, father and brother are alive',* in the first extraction (first status search) the status (keyword) 'alive' is associated only to brother, but in the second iteration steps may be implemented to associate the status (keyword) 'alive with the family members (role keywords) "mother" and "father" as well. The final list of family roles with their associated status is outputted to the next step.

Step S55

Step S55 comprises identifying at least one observation associated with a family member role keyword by, the family member role keyword being a (first) subject family member role keyword: analyzing tokens occurring in the unstructured training (medical history) data between the appearance of the (first) subject family member role keyword and the next appearance of a family member role keyword and identifying at least one token among those tokens that fulfils observation criteria as at least one observation associated with the subject family member role keyword. For example, considering the syntax tree dependencies (and POS tags), to identify observations the method needs to find nouns (POS=NOUN) and filter by the syntax tag of 'pobj' (preposition of object) or 'dobj' (direct object) or 'conj' (conjunct). A token may be considered an observation only if the tag of syntax tree dependency is inside one of those values.

Step S55 may further comprise, for another family member role keyword being a second subject family member role keyword occurring in the unstructured training (medical history) data before the appearance of the first subject family member role keyword and separated from the first subject family member role keyword by a conjunctive element (being a comma or the word "and"), identifying the at least one token which was identified as the at least one observation associated with the first subject family member role keyword as at least one observation associated with the second subject family member role keyword.

That is, step S55 comprises extracting observations associated with each family role. The syntax tree parser is used to identify observations based on their dependency with the nouns detected in the sentence. A search for observations is iterated over all the detected nouns of the text. The approach followed is similar to the first status search and the second status search of step S54. The tokens (or nouns) included between two appearances of family roles (keywords) in the text are analysed. If such tokens/nouns fulfil a set of filters and dependency types (observation criteria), they are marked as observations and associated with the first family role (keyword) appearance (i.e. the family role keyword appearing before the observation). For cases of conjunctive phrases, the same methodology used in the status extraction (second status search) is used in the observation extraction. The updated list of family roles (each family role with the related observations included) is outputted to the next step.

Steps S52 to S55 (and optionally step S56) may be considered to comprise extracting at least one group comprising (or a plurality of groups each comprising) a family member role keyword and at least one of a status keyword associated with the family member role keyword and an observation associated with the family member role keyword, wherein for any token comprising a family member role keyword and a status keyword (according to a dictionary of status keywords), the group including the family member role keyword includes the status keyword. That is, each of at least one family role keyword may be extracted together with a status keyword and/or at least one observation. They may be extracted as a token.

Step S56

Step S56 comprises extracting the modality of an observation. Step S56 may be considered as an extension of step S55, so that identifying at least one observation associated with a family member role keyword includes identifying a modality of the at least one observation, and wherein the group including the family member role keyword and the at least one observation includes the modality.

That is, step S56 comprises extracting the modality of observations (pos, neg). If a family member (role keyword) in the text is described positively with the condition/disease/symptom of the observation, this may be indicated with a positive tag (pos), and if a family member (role keyword) in the text is described negatively, i.e. without, the condition/disease/symptom of the observation this may be indicated with a negative tag (neg). External models may be used to determine if an observation is affirmative (pos tag) or negative (neg tag). The list of observations may be updated with these tags associated with the observations and outputted to the next step.

Steps S51 to S56 may be considered to comprise extracting information from the unstructured training (medical history) data for at least one family member of the target patient, the information comprising: a family member role (keyword) indicating a relationship of the family member with the target patient; a status of the family member (including at least one of a group comprising alive, dead, and healthy); and at least one observation of the family member.

Steps S51 to S56 may be considered to comprise dividing the unstructured training (medical history) data into sentences and tokenizing each sentence to extract tokens; performing noun detection among the tokens; identifying family member role keywords among the detected nouns (by comparing at least some of the detected nouns with a dictionary of family member role keywords) and storing the family member role keywords as part of an annotation dataset; identifying among the tokens at least one status keyword associated with a family member role keyword (by using a dictionary of status keywords) and storing the at least one status keyword in association with the family member role keyword in the annotation dataset; identifying among the tokens at least one observation associated with a family member role keyword and storing the at least one observation in association with the family member role keyword in the annotation dataset; using the (entities and relations in the) annotation dataset to annotate the unstructured training (medical history) data; and using the annotations in the unstructured training (medical history) data as ground truth information (and using at least one trained language model,) training the family medical history model to use NER and RE to extract as entities, from the unstructured training (medical history) data, the information (entities and relations) included in the annotations.

That is, in some implementations, rather than extracting entities as a group (entities being any of family member role keywords, status keywords, observations, family side keywords, etc.) methods may be considered to comprise extracting entities and storing them in the annotation dataset in association with each other.

Step S57

Step S57 comprises using the extracted groups (annotation dataset) to annotate the unstructured training (medical history) data. That is, step S57 comprises building an annotated dataset in JSON (JavaScript Object Notation) from the extracted entities and relations, i.e. the groups of family member role keywords and statuses/observations etc. Step S57 may comprise, after processing input Family History texts with previous steps, using external libraries for writing the information extracted in JSON format. For instance, in the text example, *"Her father died of leukemia at age 53 and her aunt had leukemia at age 19. Sister died of leukemia, Brother died from HIV/AIDS"*, the final output in JSON after previous steps and step S57 is:

"{""info"":      [{""family_role"":
{""name"": ""father""}, ""family_status"":
{""name"": ""died""}, ""family_observation"":
[{""name"": ""leukemia"", ""mod"": ""pos""}]},
{""family_role"":
{""name"": ""aunt""}, ""family_observation"":
[{""name"": ""leukemia"", ""mod"": ""pos""}]},
{""family_role"": {""name"": ""Sister""}, ""family_status"":
{""name"": ""died""}, ""family_observation"":
[{""name"": ""leukemia"", ""mod"": ""pos""}]},
{""family_role"":
{""name"": ""Brother""}, ""family_status"":
{""name"": ""died""}, ""family_observation"":
[{""name"": ""HIV/AIDS"", ""mod"": ""pos""}]}]}"

To create an annotated dataset, entities (any of the information extracted above, including family member role keywords, status keywords, observations, family side keywords, observation modalities) in the unstructured training data (family history texts) may be tagged with internal tags in the JSON format (e.g. as above). An annotation tag scheme able to represent the Named Entities and their Relationships at once may be used, so that the FMH model extracts everything (all the required entities and relations) at the same time (in one go).

In the annotation tag scheme, representation of the entities (NER) may be as follows: Family member=FH_ROLE, Family side=FH_SIDE, Family status=FH_STATUS, Family observation=FH_OBS (also optionally including a modality for each observation: FH_OBS_POS, FH_OBS_NEG). A modality may be extracted for status (FH_STATUS_POS, FH_STATUS_NEG) since or example the terms "not alive" or "not healthy" may be used. Modalities may be extracted for any of the extracted terms (family role, family side). In some implementations only a negative modality may be used and the absence of a negative modality therefore indicates a positive modality.

In the annotation tag scheme, representation of the relations (RE) may be as follows: A numeric indicator (relation) may be included at the end of a generic tag. For instance, the first appearance of a family member role keyword in a sentence (FH_ROLE_1) has a '_1' suffix. A '_1' suffix is then included in the related entities to illustrate the relation (e.g. FH_STATUS_1, FH_OBS_1, etc.). Similar suffixes may then be used for second ('_2'), third ('_3') and so on appearances of family member roles in the same sentence. This tagging scheme may also be used for nested relations, i.e. entity relations shared by various family members (e.g. FH_OBS_1_2, FH_SIDE_2_3, etc.). With this kind of representation, in a particular example of training data there are 132 tag labels but this number may depend on the training data.

For the text example, *"Mother and grandmother died of diabetes."*, the resultant annotated sentence following the annotation tag scheme is:

     <FH_ROLE_1>Mother</FH_ROLE_1> and
     <FH_ROLE_2>grandmother</FH_ROLE_2>

     <FH_STATUS_1><FH_STATUS_2>died</FH_STATUS_2></FH_STATUS_1>      of
     <FH_OBS_1><FH_OBS_2>diabetes</FH_OBS_2></FH_OBS_1>.

The annotated dataset following the annotation tag scheme is outputted to the next step.

Step S58

Step S58 comprises transforming the annotated dataset to the BIO (Begin entity, Inside entity, Other) format. That is, for the training of the FMH model (in particular the fine-tuning of the tasks of NER and Relation Extraction in the creation of the FMH model using the pre-trained language models of mBERT and BioBERT), a specific tag scheme format is required. BERT stands for Bidirectional Encoder Representations from Transformers. mBERT stands for multilingual BERT and is described at https://github.com/google-research/bert/blob/master/multilingual.md. BioBERT (an implementation of BERT that has been trained on different combinations of biomedical domain corpora) is described at https://arxiv.org/abs/1901.08746.

In the BIO format, every word of an entity is tagged. The previously annotated dataset (in JSON) is transformed to the BIO format through text processing techniques, joining and cleaning terms, and/or combining tags for nested annotations. The result of this transformation on the previous example is:

| | |
|---|---|
| Mother | B-FH_ROLE_1 |
| and | O |
| grandmother | B-FH_ROLE_2 |
| died | B-FH_STATUS_1_2 |
| of | O |
| diabetes. | B-FH_OBS_1_2 |

The annotated dataset transformed to this BIO format will be the final output of the Label Annotator Engine in the running example. BIO / IOB format (short for inside, outside (other), beginning) is a tagging format for tagging tokens in a chunking task in computational linguistics. The B- prefix before a tag indicates that the tag is the beginning of a chunk, and an I- prefix before a tag indicates that the tag is inside a chunk. An O tag indicates that a token belongs to no entity/chunk. There are other formats that could be used, e.g. BIOES (BIO is the same + E- prefix before a tag indicates that the tag is the end of a chunk, and an S-prefix before a tag indicates that the chunk is single (single element=one word). BIOES is also called BILOU (L for last, same as the E, and, U for unique, same as the S).

In some implementations/examples, some method steps or parts of method steps may be omitted and/or combined and/or executed in a different order. For example steps S52-S56 may be considered as a single step comprising some or all of those steps, i.e. of extracting a group (of words/tokens/entities) comprising a family member role keyword and at least one of a status keyword associated with the family member role keyword and an observation associated with the family member role keyword, wherein for any token comprising a family member role keyword and a status keyword (according to a dictionary of status keywords), the group including the family member role keyword includes the status keyword.

A family medical history (FMH) trainer trains the FMH model using the annotated unstructured training data output from the label annotator engine. That is, the FMH trainer is in charge of implementing a deep learning process, for example through state-of-the-art techniques and third-party tools, using the annotated dataset created with the Label Annotator Engine. For example, the external libraries of Tensorflow (https://github.com/tensorflow/tensorflow) and Keras (https://keras.io/) for Python may be used to train the FMH model.

The inputs to the FMH trainer are the annotated dataset from the label annotator engine and e.g. the pre-trained language models mBERT and BioBERT.

The FMH trainer implements techniques for fine-tuning NER and Relation Extraction in Family Medial History data (unstructured (medical history) data) using pre-trained language models. The operations of the FMH trainer include methods of pre-processing and preparation of the input annotated training dataset, conversion of texts to numerical representations, design of the neural network architecture and execution of the deep learning activity.

The output of the FMH trainer is a trained Family Medical History (FMH) model which is capable of extracting family medical information from new unstructured clinical documents. This model is used to extract family history information from the input data.

A Family Medical History (FMH) Extractor processes the input data using the trained FMH model (trained previously). The trained FMH model may be considered to perform a prediction over the patient's data, returning the results in the BIO format defined during the training of the FMH model. Next, the FMH Extractor transforms the data from the BIO format/scheme to a JSON data structure containing for each identified family member of the patient:

   1. Family member role: The role of the family member in relation to the target/current patient. Also, in this step, the extracted family member roles may be analysed to identify inner-relations between them - although the Genogram is based on one main patient (the target patient), it may include relations between other family members.
   2. Family member status: Current status of the family member (e.g. alive, dead, healthy, etc.)
   3. Family member side: Side of the family member (maternal or paternal)
   4. Family member observations: A list containing details of the observation(s) (diseases, affections, conditions, symptoms, etc.) of the family member
   5. Family member observation modality: Tagging the observation as positive (family member has the disease, affection, condition, symptom, etc), or negative (family member does not have the disease, affection, condition, symptom, etc).

This is a generic example of the output of the FMH Extractor:
{'info': [{'family_role': {'name': 'father', 'mod': 'pos'}, 'family_status': {'name': 'died', 'mod': 'pos'}, 'family_observation': [{'name': 'a brain tumor', 'mod': 'pos'}]}, {'family_role': {'name': 'mother', 'mod': 'pos'}, 'family_status': {'name': 'died', 'mod': 'pos'}, 'family_observation': [{'name': 'diabetes complications', 'mod': 'pos'}]}], 'text': 'Father died of a brain tumor. Mother died of diabetes complications.'}

This JSON output is then processed. The family member roles are analysed to obtain inner-relations between them using a rule-based approach over known family relations.

Then, the data is for example included in a table with headings: "family member 1"; "relationship"; "family member 2"; "status"; "observation".

To populate this table, the JSON output is processed and special identifiers are added to names of family members to make them unique in the data to avoid different-person and same-name ambiguities. The table then contains the extracted family history information. The format of the information may not be a table format or a JSON format. Any suitable format may be used to record/store the information.

Medical Named Entity Recognition (NER) extraction

NER is used to extract medical entities from the unstructured data, and therefore to extract medical history information. The methodology described in a paper titled "FLE at CLEF eHealth 2020: Text Mining and Semantic Knowledge for Automated Clinical Encoding" by Nuria Garcia-Santa and Kendrick Cetina (available at http://ceur-ws.org/Vol-2696/paper_111.pdf) may be used to extract the Named Entities from the unstructured data.

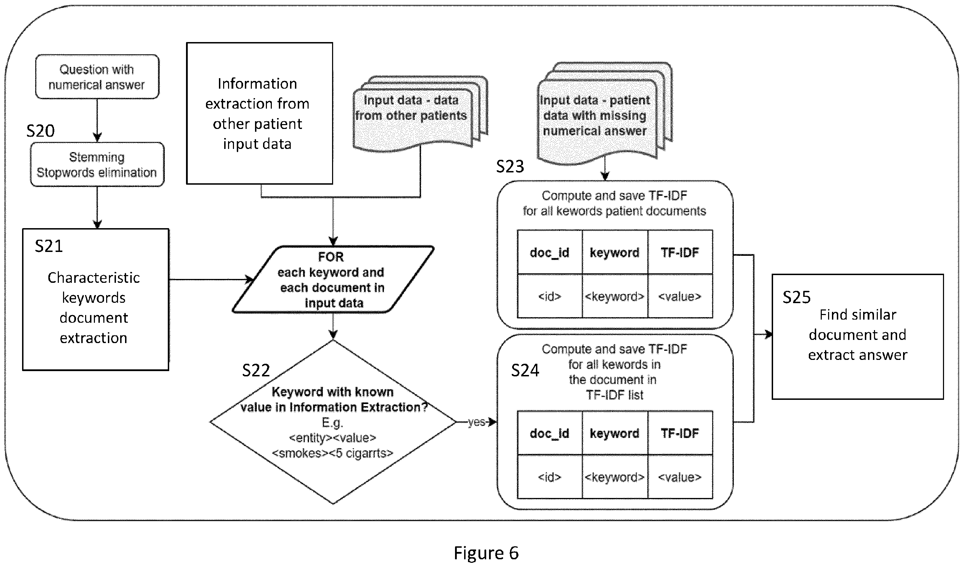

Figure 6 is a diagram illustrating a method employing the numerical answer prediction model to predict a numerical answer to a question. The method in Figure 6 may be implemented by a module which may be referred to as module: numerical answer prediction model. The method illustrated in Figure 6 may be implemented by the answer manager, or by both the answer manager and the module: numerical answer prediction model. The method illustrated in Figure 6 may be referred to as a numerical answer generation flow.

The numerical answer generation flow may be summarized for a given question as:

> If the answer to the question is found in the Extracted Information from Module: Information Extraction
> Then return answer
> Else: Use Numerical Answer prediction model to find an answer

The numerical answer generation flow may be considered to comprise only the prediction of a numerical answer, the other steps of the above summarization being carried out in the answer generation flow described with reference to Figure 1. The numerical answer generation flow may be considered to comprise steps S20 to S25 in Figure 6.

The method of Figure 6 is carried out when an answer to a numerical question is not found in the extracted information (in step S12). The method in Figure 6 will now be described in more detail.

As an overview, a numerical answer is predicted by searching for a numerical answer in another, similar document. Documents related to other patients are filtered by question keywords. In this way documents likely to contain answers related to the numerical answer are obtained. Next, a measure of similarity between the document of the target patient and documents of other patients is used to find similar documents and to associate a known numerical answer to the target patient. The heterogeneity of documents from other patients means that a robust (overfitting resistant) way to assess the similarity between patients is useful. This is done in a running example by reducing the number of similarity parameters to the (question) keywords in the documents and it has been found through experiments that, among multiple similarity measures, the measure for keyword-wise similarity provided by TF-IDF scores (described below) has the best performance for this problem.

Some assumptions for the approach of the running example to achieve a good performance may be (but are not necessary):

- that there is a big enough pool of documents from external patients
- that there is a closed set of possible numerical answers, e. g., questions like "how many days a week...?" limits the possible answers to the whole numbers between 0 and 7.
- that patients with similar habits (numerical answers) yield similar documents.

Step S20 - Stemming and stop-words removal

The question is cleaned with the following NLP techniques:

1. Stemming

Stemming usually refers to a heuristic process that involves removing the ends of words, and often includes the removal of derivational affixes. The goal of stemming is to find the root or stem of a word so that variations on that stem (which may be referred to as inflected forms) can be more easily identified/processed. Stemming may be carried out using a lookup table of a stem/root word and its inflected forms. Stemming may be carried out using rules such as "if the word ends in 'ed', remove 'ed'". Such rules may be aimed at removing suffixes. Here are two examples of stemming:

"changing", "changed", and "change" will, after stemming, become "chang".

"studying", "studies", and "study" will, after stemming, become "stud".

2. Stop-words removal

Stop-words removal is an NLP procedure that comprises the removal of words that are considered to add noise to NLP learning because of the high frequency of these words appearing in any given language. These words are usually articles, prepositions, pronouns, conjunctions, etc. and usually do not add much information to the text. Examples of a few stop words in English are "the", "a", "an", "so", "what". Stop-words removal may be carried out by searching for stop-words defined in a dictionary or set of stop-words and removing from the question any identified stop-words.

The words remaining after stemming and stop-word removal have been performed on a question are extracted as characteristic keywords for that question.

In other words, step S10 comprises cleaning the question using stemming and stop-words removal and extracting the remaining words as characteristic keywords, wherein stemming comprises removing at least one character from the end of at least one word of the question to obtain the stem/root of the word, and wherein stop-words removal comprises identifying stop-words in the question using a set/dictionary of stop-words and removing the identified stop-words.

Step S11 - Characteristic keyword document extraction

Step S11 comprises extracting relevant documents from input data and from other input data, the other input data comprising (unstructured) text data relating to at least one patient other than the target patient (and e.g. not relating to the target patient). The other input data may be referred to as further unstructured data.

For a given question, the characteristic keywords in the question are used to find the relevant documents in the Input data and the other input data. By extracting relevant documents, the search for an answer (described later below) may be limited to the relevant documents and corresponding relevant rows in the Extracted information.

To perform characteristic keyword document extraction in a particular example, an NLP algorithm Latent Dirichlet Allocation (LDA) that can be found in libraries like Gensim (https://pypi.org/project/gensim/) is used. LDA is a generative unsupervised probabilistic algorithm that isolates the top K (K being a positive integer) topics in a dataset as described by the most relevant N (N being a positive integer) keywords. In other words, the documents in the data set (e.g. further input data) are represented as random mixtures of latent topics, where each topic is characterized by a Dirichlet distribution over a fixed vocabulary. "Latent" means that topics are inferred in the documents and do not need to be directly observed. Other methods may be used to perform characteristic keyword document extraction, for example a search within the text of each document for words/terms related to the characteristic keywords and/or topics.

Information extraction is carried out on the other input data (referred to as other patient input data or "input data - data from other patients" in Figure 6) to generate other extracted information or further extracted information (as opposed to the "extracted information" from information extraction performed on the input data). The methods for information extraction from the other input data are the same or similar as those for the input data described above and duplicate description is omitted.

Term frequency-inverse document frequency (TF-IDF) computation

TF-IDF for a word in a document is calculated by multiplying two measures:

1. The term frequency of a word in a document. There are several ways of calculating this frequency, the simplest being a raw count of instances the word in the document. There are ways to adjust the frequency, for example by dividing by the length of the document (e.g. in words), or by the raw frequency of the most frequent word in a document. The measure may be further adjusted by computing the logarithm of (1+the frequency).

2. The inverse document frequency of the word across a set of documents. This quantity is a measure of how common or rare the word is across the document set. The closer this measure is to 0, the more common the word is. This measure can be calculated by taking the total number of documents, dividing it by the number of documents that contain the word, and calculating the logarithm of the result.

The TF-IDF score for the word t in the document d from the document set D is calculated as follows in a running example:

$$tf\ idf\ (t, d, D) = tf\ (t, d) \cdot idf\ (t, D)$$

where

$$tf\ (t, d) = log\ (1 + freq\ (t, d))$$

$$idf\ (t, D) = log\ (N\ /\ (count\ (d \in D: t \in d))$$

The higher the TF-IDF for a word in a document, the more relevant that word is in that particular document.

The TF-IDF is an example of a relevance measure. Other quantities may be used as the relevance measure, for example the term frequency.

In step S23, relevance measures are computed for the input data which relates to the target patient and which does not include an answer to the question under consideration. In step S24, relevance measures are computed for the other input data which relates to other patients. That is, relevance measures are computed using two different sets of documents generating one table of TF-IDF scores (relevance measures) for each set of documents. The other input data may be publicly available data from patients and may include information corresponding to a numerical answer to the question under consideration.

Steps S23 and S24 may be summarized as follows:

For each document d of the set D:

For each characterising keyword t in the list of characterising keywords extracted from the question:

Compute TF-IDF score of t in d

In step S23 the set D of documents is the set of documents in the input data relating to the target patient. In step S24 the set D of documents is the set of documents in the other input data relating to other patients. In a running example, the set D of documents for use in step S23 and the set D of documents for use in step S24 are limited to documents deemed relevant in step S21. The set D of documents for use in step S24 may be further limited by step S22 as shown in Figure 6. In step S22 it is determined which documents from the other input data include a numerical answer to the question under consideration. This is determined by analysing the other extracted information which has been extracted from the other input data (in a way corresponding to how a numerical answer is selected from the extracted information described above with reference to step S13 of Figure 1). Step S24 may therefore be implemented only using documents from the other input data that include an answer to the question under consideration.

A relevance measure of a characteristic keyword in a document may be considered to be a relevance measure of that characteristic keyword in that document compared to a set of or all documents in the input data/other input data.

In other words, steps S23 and S24 comprise: computing, for each of the characteristic keywords extracted from the question, a relevance measure of the characteristic keyword in a document from the unstructured data relating to the target patient (compared to all/a set of documents in the unstructured data relating to a target patient); and computing, for each of the characteristic keywords extracted from the question, a relevance measure of the characteristic keyword in each of a plurality of documents relating to other patients (compared to the plurality of or a set of the documents relating to the other patients).

The relevance measures may be stored as shown in Figure 6, steps S23 and S24, storing each relevance measure in association with the document ID of the given document and the characteristic keyword.

Using the tables including the document id and the keyword from which the TF-IDF was generated, a similar document among the other input data is determined, as described below. The steps S20 to S24 may be carried out for a plurality (or all) of the numerical questions for which an answer could not be extracted from the input data. The steps S20 to S24 may be carried out for all these questions one at a time, each time followed by step S25 described below. The steps S20 to S24 may be carried out for all these questions before step S25.

Step S25 - Similarity finding

To predict an answer to a given numerical question, a search for a document in the other input data similar (by keywords and TF-IDF values) to a document in the input data is performed.

If the steps S20 to S24 have been carried out for multiple numerical questions and the tables relate to characteristic keywords from multiple questions, then the rows in the TF-IDF tables that contain the characteristic keywords from the question currently under consideration are selected. This isolates the documents (corresponding to the rows e.g. by the document ID field) that contain information that may be used to generate the answer. If the tables relate only to characteristic keywords from the question currently under consideration then the above selection of rows is not carried out.

If relevance measures have been computed for multiple documents of the input data relating to the target patient then one of those documents may be selected for the comparison step described below. The document selected may be the document that contains the most of the different characteristic keywords from the question under consideration among the documents, or the document with the highest relevance measure for at least one of the characteristic keywords, or a mixture of both these considerations.

The relevance measures for the selected document of the input data are compared with the relevance measures for the documents (of the selected rows if rows have been selected) of the other input data. The document from the other input data with relevance measures (TF-IDF scores) most similar to those of the selected document of the input data is identified and other extracted information from the identified document is selected/stored as the answer to the numerical question under consideration.

Identifying one of the plurality of documents relating to the other patients in which the relevance measure of the at least one characteristic keyword is most similar to the relevance measure of the at least one characteristic keyword, (respectively), in the document in the unstructured data relating to the target patient may comprise, when the at least one characteristic keyword comprises a plurality of characteristic keywords

- for each of the plurality of documents relating to the other patients and for each characteristic keyword, assigning a similarity score to the relevance measure of the characteristic keyword in the document of the plurality of documents relating to the other patients depending on how similar the relevance measure is to the relevance measure of the characteristic keyword in the document in the unstructured data relating to the target patient;
- for each of the plurality of documents relating to the other patients, aggregating the similarity scores for the document; and
- identifying the document with the highest similarity score.

Assigning a similarity score to the relevance measure of the characteristic keyword in the document of the plurality of documents relating to the other patients depending on how similar the relevance measure is to the relevance measure of the characteristic keyword in the document in the unstructured data relating to the target patient may comprise

- assigning a first value as the similarity score when a difference between the relevance measure of the characteristic keyword in the document of the plurality of documents relating to the other patients and the relevance measure of the characteristic keyword in the document in the unstructured data relating to the target patient is less than a threshold difference; and
- assigning a second value as the similarity score when a difference between the relevance measure of the characteristic keyword in the document of the plurality of documents relating to the other patients and the relevance measure of the characteristic keyword in the document in the unstructured data relating to the target patient is more than or equal to the threshold difference.

If the first value is higher than the second value then a higher overall similarity score indicates higher similarity, but if the first value is lower than the second value then a lower overall similarity score indicates higher similarity.

The method may use multiple threshold differences to assign multiple different values depending on how similar the two relevance measures under comparison are.

Relevance measures do not need to be computed for all characteristic keywords in a given question. The method steps in Figure 6 may be carried out in a different order and some steps may be omitted. Each step has been described according to a particular running example. The scope of the invention is defined in the claims.

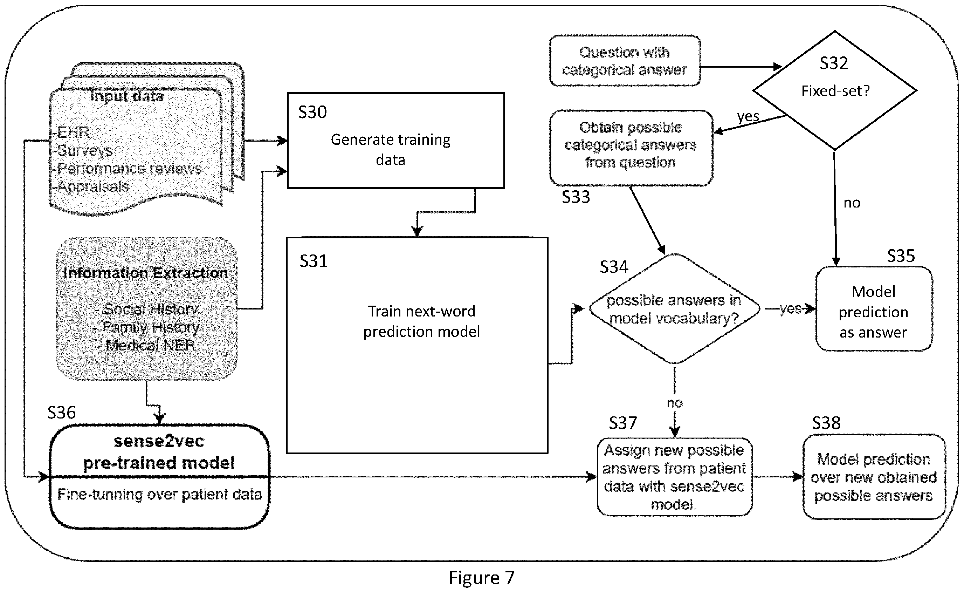

Figure 7 is a diagram illustrating a method employing the categorical answer prediction model to predict a categorical answer to a question. The method in Figure 7 may be implemented by a module which may be referred to as module: categorical answer prediction model. The method illustrated in Figure 7 may be implemented by the answer manager, or by both the answer manager and the module: categorical answer prediction model. The method illustrated in Figure 7 may be referred to as a categorical answer generation flow.

The categorical answer generation flow may be summarized for a given question as:

> If the answer to the question is found in the Extracted Information from Module: Information Extraction
> Then return answer
>
> Else: Use Categorical Answer prediction model and Answer-value-type categorization (open/fixed set) information to find an answer:
>
> > If the Answer-value-type is open
> > then return a next-word prediction model's next-word prediction as answer
> >
> > If the Answer-value-type is fixed set and fixed set of possible answers are in model vocabulary then return the possible answer that has maximum prediction probability outputted from the next-word prediction model.
> >
> > If the Answer-value-type is fixed set and fixed set of possible answers are not in model vocabulary
> > Then find possible answer replacements (with sense2vec model) and return the answer that has maximum prediction probability outputted from the next-word prediction model.

The categorical answer generation flow may be considered to not comprise "If the answer to the question is found in the Extracted Information from Module: Information Extraction: Then return answer". This may be carried out in the answer generation flow described with reference to Figure 1. The categorical answer generation flow may be considered to comprise steps S30 to S38 in Figure 7.

The method of Figure 7 is carried out when an answer to a categorical question is not found in the extracted information (in step S12). The method in Figure 7 will now be described in more detail.

Next-Word Prediction Model

The next-word prediction model is a model that considers a sequence of words and predicts the next word in the sequence. The model employs natural language processing and deep learning. The model is used by the categorical answer prediction model to generate as an answer to a question a predicted subject next word based on a subject sequence of words in in the question as input/as a seed. The model may predict multiple preliminary subject next words and return the probability that each preliminary subject next word is correct (or the probability of each word in its vocabulary to be the subject-next word or answer).

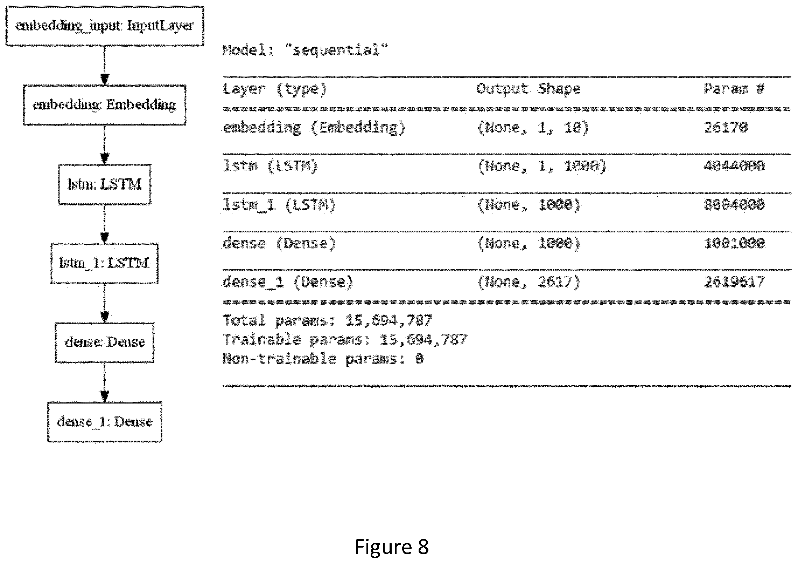

In a particular example, the next-word prediction model is a machine-learning sequential model generated by starting with an initial embedding layer, then adding a Long short-term memory, LSTM (which is an artificial recurrent neural network (RNN) architecture used in the field of deep learning) with 1000 units that will be passed through another LSTM layer with 1000 units, to be passed through a hidden layer with 1000 node units using a dense layer function with Rectified Linear Unit (relu) set as the activation. The last layer is a softmax activation. The softmax activation ensures that probabilities for the outputs (preliminary subject next words) are equal to the vocabulary size (in a set of data (text) the vocabulary size is the number of unique words in the data). The definition of the architecture and a simplified view of the layers is illustrated in Figure 8. Other architectures may of course be used.

The next-word prediction is trained before use. This training and the generation of training data for that purpose (steps S30 and S31) may or may not be considered part of the categorical answer generation flow and may be carried out once at the start of the flow and not for each question for which a categorical answer is predicted. The training may be carried out once for a particular set of input data and for example may be carried out again if different input data is used (i.e. when computing a health metric for a new target patient).

Step S30 - Training data assembly/generation for Next-word prediction model

Step S30 comprises generating training data for training the next-word prediction model. The training data is a dataset comprising input sequences and corresponding predictions. The training data is generated based on the input data and the extracted information.

The training data is assembled by extracting sequences of words from the input data and/or from the constituent sentences in the extracted information and removing the last word from each sequence to generate training sequences of words. The removed last word corresponding to a training sequence is used as ground truth data when training the model (step S31). For example, given the sequence of words "Patient is a smoker since 2010" the training sequence is: "Patient is a smoker since" and the corresponding (correct) prediction (ground truth data) is "2010". Tokenization of sentences may be employed in step S30. That is, the process of separating the last word of a sentence from the rest of the sentence may be considered and implemented as a type of tokenization.

Sequences of words may be extracted from throughout the input data and/or from each constituent sentence.

Step S31 comprises training the next-word prediction model using the training data. This may be an iterative process of adjusting the model (including weights of nodes, etc.) until the model correctly predicts a certain percentage of next words, for example. The training may be performed by expressing words as numerical arrays (embeddings) that contain relevant information like context and meaning of words.

In other words, steps S30 and S31 may be considered to comprise assembling training data by extracting sequences of words from the unstructured data and/or from the constituent sentences in the extracted information, removing the last word of each sequence to generate training sequences of words; and training the neural network/model to predict the next word in each training sequence of words, using for each training sequence of words the removed last word as ground-truth data.

Step S32 comprises, for a given categorical question, determining whether the question is a fixed-set question or not, for example based on the classification assigned in step S11. If the question is fixed-set (yes) the method proceeds to step S33, and if the question is not fixed-set (no) the method proceeds to step S35. Step S35 comprises a model prediction. For example, in the case that the question is not a fixed-set question, step S35 comprises using the trained next-word prediction model to predict the subject next word using a subject sequence of words extracted from the question under consideration and selecting/storing the subject next word as the answer. The next-word prediction model may predict a plurality of preliminary subject next words and the one with the highest probability (also output by the next-word prediction model) of being the correct next word may then be selected as the answer.

Step S33 comprises obtaining the possible answers for the question, for example by obtaining the possible answers extracted in the step S11.

Step S34, following step S33, comprises using the trained next-word prediction model to predict the subject next word or multiple preliminary subject next words using a subject sequence of words extracted from the question under consideration. Step S34 further comprises determining whether the predicted subject next word matches one of the possible answers, or if one of the preliminary subject next words (with a probability above a threshold probability and/or in the top M (M being a positive integer) preliminary subject next words sorted by probability) matches one of the possible answers.

If the determination in step S34 is "yes", the method proceeds to step S35 and the predicted subject next word is selected/stored as the answer, or (in the case of multiple preliminary subject next words) the matching preliminary subject next word is selected as the predicted subject next word which is then selected/stored as the answer.

If the determination in step S34 is "no", the method proceeds to step S37.

Instead of using the next-word prediction model to generate a subject next word or preliminary subject next words, the vocabulary of the next-word prediction model could instead be examined to determine whether (at least one of) the possible answers (is) are in the model's vocabulary.

That is, the ability of the next-word prediction model to generate predictions depends on the vocabulary of the training data (the model vocabulary). This means the model can only predict words that are in its vocabulary. This limits the model in situations where the fixed-set of possible answers contains words that are not in the vocabulary in the model.

To alleviate this issue, words related to the possible answers not in the model vocabulary may be found that are in the model vocabulary. Such related words may be found using the sense2vec pre-trained model, fine-tuned with the extracted information (i.e. by performing further training of the sense2vec model with some extracted information as training data.. Sense2vec is a word2vec-based embedding that allows the exploration of semantic similarities. Sense2vec is publicly available (https://github.com/explosion/sense2vec) and can be used to carry out the replacement of not-found words with words or terms that convey the same or similar meaning.

Step S36 of the model illustrated in Figure 7 and according to a running example comprises fine-tuning the sense2vec model using the extracted information.

Step S37 comprises assigning replacement (new) possible answers based in the running example on the sense2vec model search. The sense2vec model (or other model that is used) may output a sorted-by-similarity list of terms (or a list of terms with corresponding term similarity scores indicating their similarity to the given possible answer to be replaced) to replace the or each possible answer and the term highest on the list (i.e. with the highest term similarity score) is selected to be assigned as the new possible answer.

Step S38 (following step S37) comprises selecting/storing a predicted subject-next word as described above with reference to step S35 but using the replacement possible answers assigned in step 37. The final answer that is selected/stored is the possible answer from the question that corresponds to the replacement possible answer selected.

Steps S37 and S38 may instead comprise comparing a predicted subject next word output in step S34 and, if/when the predicted subject next word is the same as one of the at least one similar words (found e.g. by the sense2vec model), selecting/storing the possible answer corresponding to the predicted subject next word as the categorical answer.

Steps S37 and S38 may instead comprise, when a plurality of preliminary subject next words have been generated in step S34, if/when one of the preliminary subject next words is the same as one of the at least one (or the top Y most similar) similar words (found e.g. by the sense2vec model), selecting that preliminary subject next word as the predicted subject next word, and then selecting storing the possible answer corresponding to the predicted subject next word as the categorical answer. The comparison of the preliminary subject next words may start with the preliminary subject next word with the highest probability and the most similar word and work down each list.

To exemplify the step S34 and a possible output of the sense2vec model, an example fixed set of possible answers for a given question may be: ['balanced diet', 'not balanced diet'] and, for example, step S34 determines that the answer "balanced diet" cannot be found in the model vocabulary. The fine-tuned Sense2Vec model lists the most similar terms to the original fixed-set answer that is not available in the next-word model vocabulary. The following list is an example of the sense2vec output for "balanced diet":

| healthy diet | 89% |
| well balanced diet | 84% |
| good diet | 81% |
| well rounded diet | 81% |
| varied diet | 80% |
| healthy balanced diet | 79% |
| healthy, balanced diet | 79% |

The method steps in Figure 7 may be carried out in a different order and some steps may be omitted. Each step has been described according to a particular running example. The scope of the invention is defined in the claims.

Worked Example: Gallup-Sharecare Well-Being Index automation

The Gallup-Sharecare Well-Being Index measures Americans' perceptions of their lives and their daily experiences through five interrelated elements that make up well-being: sense of purpose, social relationships, financial security, relationship to community, and physical health.

In this example the Gallup-Sharecare wellbeing index will be automatically computed. The Wellbeing Index data and questionnaire may be obtained from publicly available sources such as: https://www.gallup.com/175196/gallup-healthways-index-methodology.aspx.

In this example, a system implementing the method receives the following inputs: wellbeing index metric data (questionnaire and rules) and input data (EHR of a target patient). The system also receives other input data relating to other patients.

Wellbeing Index Metric Data: Questionnaire

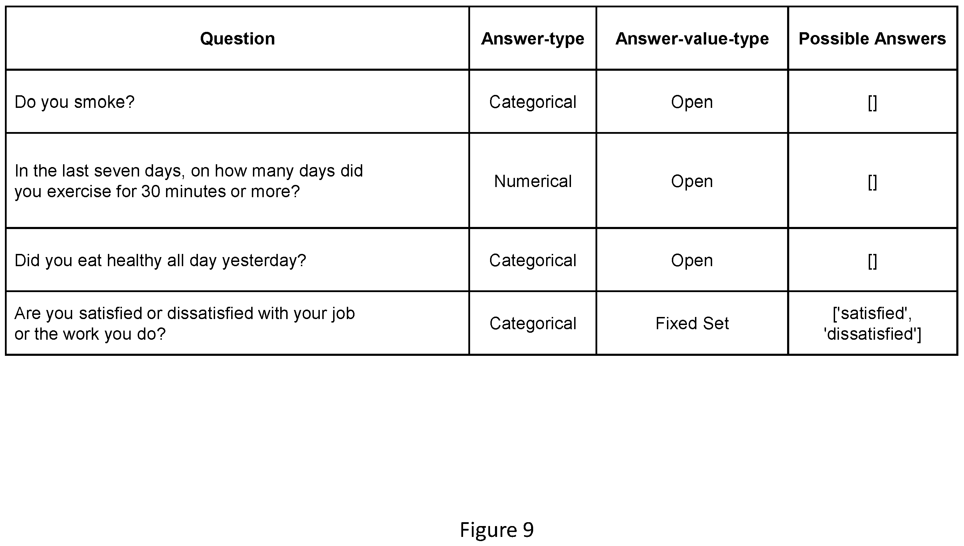

The following is an excerpt of the questionnaire needed to compute the wellbeing index: https://news.gallup.com/file/poll/146822/Healthways_UKWellbeing_Questions_en-UK_03-17-11_td.pdf
For convenience in the description of this example four questions from the questionnaire are considered. These four questions are shown in the table in Figure 9. Steps S10 and S11 are carried out on the four questions to generate the information shown in Figure 9, namely the answer-type and answer-value type for each question, and the possible answers (if the question is a fixed-set question).

Input Data: EHR of test patient.

The following text is an excerpt of an EHR found in the publicly available source, https://mtsamples.com/site/pages/sample.asp?Type=97-Consult%20-%20History%20and%20Phy.&Sample=1954-Bilateral%20Hip%20Pain

The patient is a 38-year-old woman presenting to our clinic for the first time for evaluation of hip pain, right greater than left, of greater than 2 years' duration. The patient states that she began with right hip pain getting steadily worse over the last 2 years and has now developed some pain in the left hip. The pain is located laterally as well as anteriorly into the groin. She states that the pain is present during activities such as walking, and she does get some painful popping and clicking in the right hip. She is here for evaluation for the first time. She sought no previous medical attention for this.
PAST MEDICAL HISTORY: Significant for depression and reflux disease. PAST SURGICAL HISTORY: Cesarean section x 2.
CURRENT MEDICATIONS: Listed in the chart and reviewed with the patient. ALLERGIES: The patient has no known drug allergies.
SOCIAL HISTORY: The patient is married. She is employed as an office manager. She does smoke cigarettes, one pack per day for the last 20 years. She consumes alcohol 3 to 5 drinks daily. She uses no illicit drugs. She exercises monthly mainly walking and low impact aerobics. She also likes to play softball.
REVIEW OF SYSTEMS: Significant for occasional indigestion and nausea as well as anxiety and depression. The remainder of the systems negative.

The above text is used as the input data for this example.

Information extraction from Input Data

In this example only one EHR from the target patient is used, but in other examples information is extracted from more than one document in the input data. The three types of information extraction are social history extraction, family history extract, and medical NER extraction.

Social history information extraction

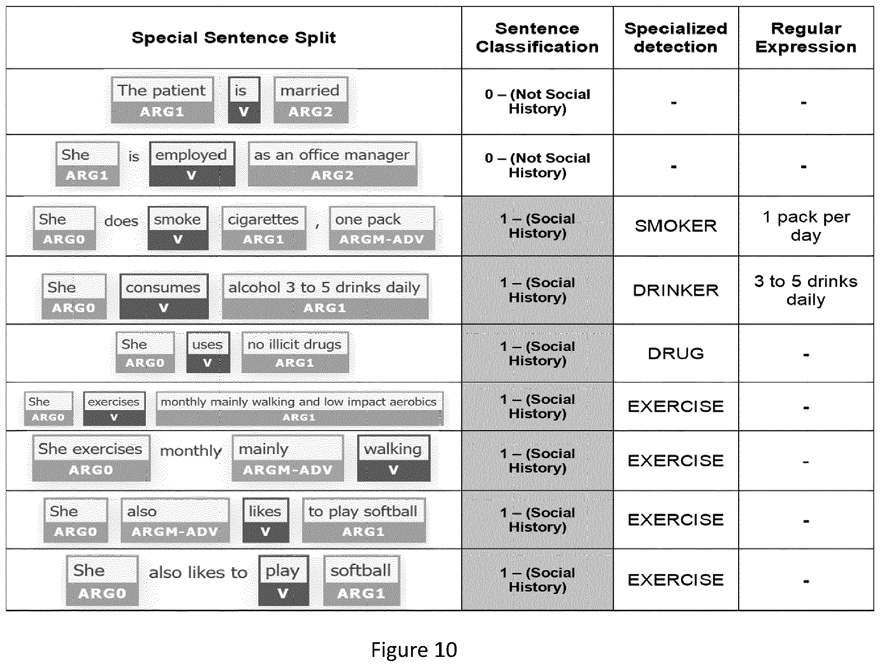

Social history information is extracted in the way described above with reference to Figure 2. The table shown in Figure 10 shows the extracted social history information. "Special sentence split" illustrates how constituent sentences are extracted by parsing a sentence. "Sentence classification" indicates whether the constituent sentence includes social history information (1) or not (0). "Specialized detection" indicates to which social history topic the information in the constituent sentence has been categorized as corresponding to. "Regular expression" indicates any numerical value in the constituent sentence and its unit of measurement.
Family History information Extraction - No Family History information was found in the input data (the EHR text extract above) using the methods described above for family history information extraction.

Medical NER extraction

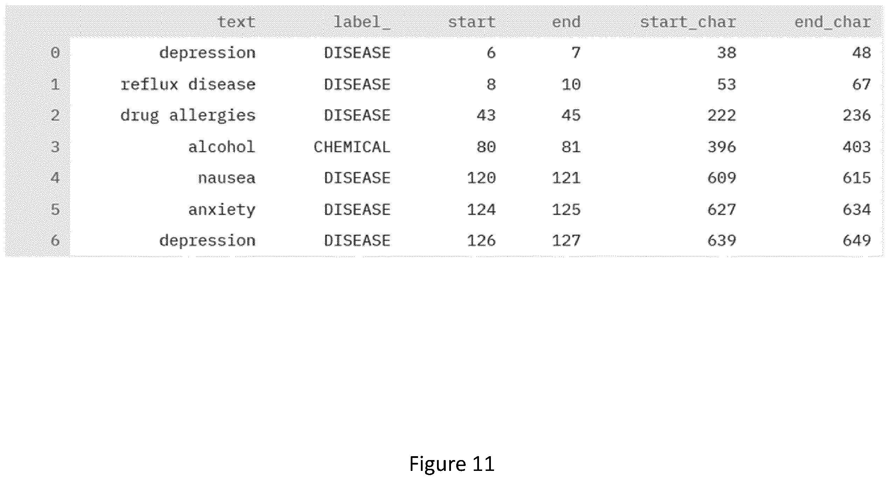

In this example the NER models DISEASE and CHEMICAL produced as described above are used. The NER extraction returns a list of all entities found in the text. These entities are shown below with annotations after the entities, and also in a table with information related to the positions of the entities in the text in Figure 11. For convenience in describing the example, only a part of the above text extract from the EHR is used.

PAST MEDICAL HISTORY: Significant for depression **{DISEASE}** and reflux disease **{DISEASE}.**
PAST SURGICAL HISTORY: Cesarean section x 2.
CURRENT MEDICATIONS: Listed in the chart and reviewed with the patient. ALLERGIES: The patient has no known drug allergies **{DISEASE}.**
SOCIAL HISTORY: The patient is married. She is employed as an office manager. She does smoke cigarettes, one pack per day for the last 20 years. She consumes alcohol **{CHEMICAL}** 3 to 5 drinks daily. She uses no illicit drugs. She exercises monthly mainly walking a low impact aerobics. She also likes to play softball. REVIEW OF SYSTEMS: Significant for occasional indigestion and nausea **{DISEASE}** as well as anxiety **{DISEASE}** and depression **{DISEASE}.** The remainder of the systems negative.

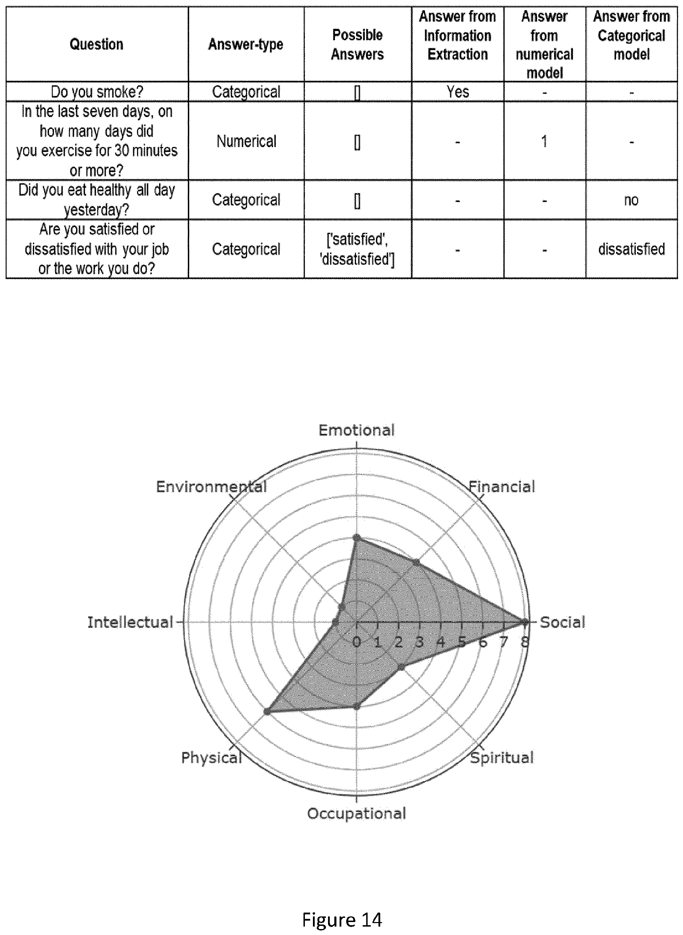

The answer generation flow is followed to extract any possible answers from the extracted information. The answer to the first question "Do you smoke?" is extracted from the extracted information in step S12/13, as shown in the first row of the table in Figure 14.

Numerical Answer prediction

The numerical answer prediction model is used to predict an answer to the question "In the last seven days, on how many days did you exercise for 30 minutes or more?"

Step S20 is carried out, including stemming, stop-words removal and characteristic keyword extraction, and the following characteristic keywords are obtained ['days', 'how', 'many', 'days', 'exercise', '30 minutes']

The following are the sentences from the EHR that contain at least one of the keywords in the question. It will be appreciated that no numerical answer to the question can be extracted from these sentences.

(**ARG0**: She) (**V**: exercises) (**ARG1**: monthly mainly walking and low impact aerobics)

(**ARG0**: She exercises) monthly (**ARGM-ADV**: mainly) (**V**: walking)

(**ARG0**: She) also likes to play softball

(**ARGO**: She) also likes to (**V**: play) (**ARG1**: softball)

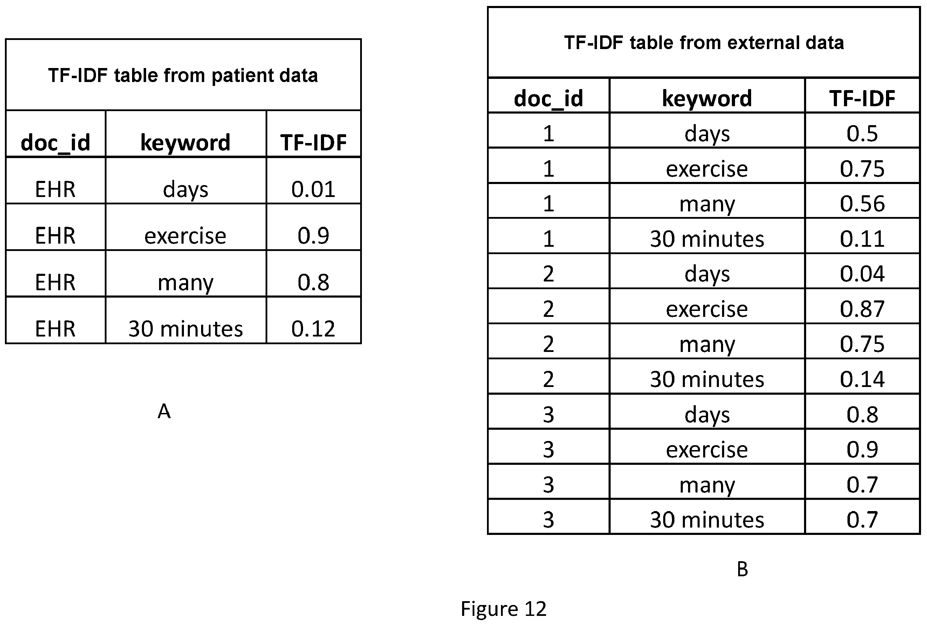

Other input data relating to other patients is used and information extraction is performed on this data. After steps S22, S23, and S24, two TF-IDF tables are obtained, one comprising TF-IDF values for characteristic keywords in the EHR extract of the input data relating to the target patient, and the other comprising TF-IDF values for characteristic keywords in documents from the other input data (or "external data") related to other patients, with known numerical value answers. The TF-IDF tables are tables A and B in Figure 12.

For convenience in describing this example, only some of the characteristic keywords are considered here. A comparison in step S25 determines that the TF-IDF values from the EHR relating to the target patient are most similar to the TF-IDF values, respectively, for document 2 from external data. Step S25 comprises extracting a numerical answer from document 2 and using this as the answer to the question under consideration. This is shown in the table in Figure 14, second row.

Categorical Answer prediction

Two categorical questions remain unanswered: "Did you eat healthy all day yesterday?"; and "Are you satisfied or dissatisfied with your job or the work you do?". The categorical answer prediction model is used to predict answers to these questions.

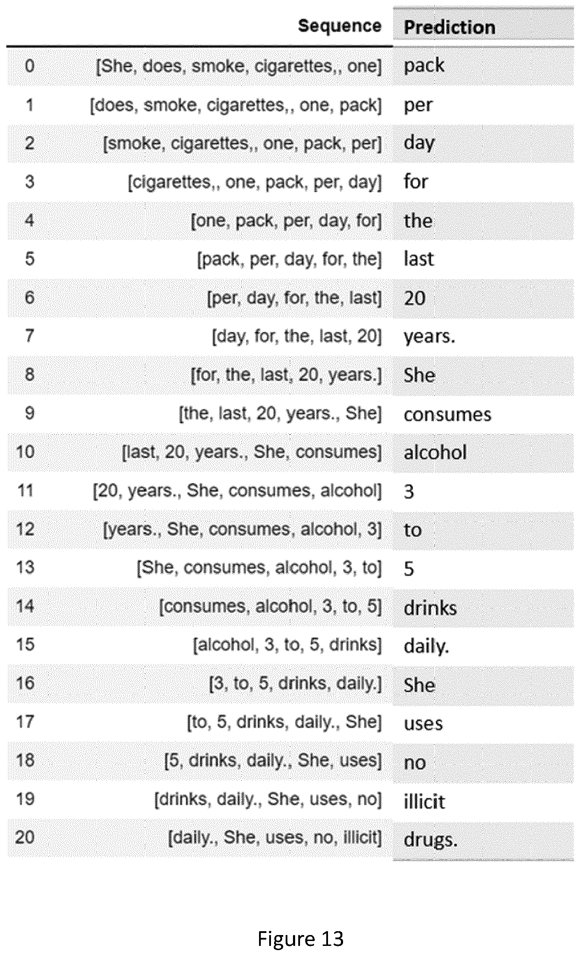

In step S30 training data is generated from the input data (from the EHR extract) and from the extracted information (constituent sentences). Sequences of text from the input data and extracted information are generated with the corresponding correct predictions being the next word of the sequence. The table in Figure 13 shows some of the generated training data (training sequence and next word as ground truth).

In step S31 a Next-Word prediction model (with the architecture described above with reference to Figure 8) is trained using the training data. Using the trained model, the remaining steps of Figure 7 are followed to predict answers to the remaining two questions

For the question "Did you eat healthy all day yesterday?", in step S32 it is determined that the question is not a fixed-set question and the method proceeds to step S35. The subject sequence (input seed) for the next-word prediction model is "eat healthy all day yesterday?". The model prediction (predicted subject next word) is "No" with probability 0.845.

For the question "Are you satisfied or dissatisfied with your job or the work you do?", in step S32 it is determined that the question is a fixed-set question and the method proceeds to step S33 in which the possible answers: ['satisfied' 'dissatisfied'] are obtained. In step S34 it is determined that the possible answers are not in the next-word prediction model's vocabulary, that is, there are no ['satisfied' 'dissatisfied'] words in the vocabulary of the model. In step S36 a sense2vec model is fine-tuned using the EHR data and lists of possible replacements for the words ['satisfied' 'dissatisfied'] are obtained. The candidate replacements for satisfied' are:

pleased 62%
happy 61%
sated 61%
content 60%
satisfactory 59%

And the candidate replacement for dissatisfied' are:

unhappy 78%
unfulfilled 72%
unsatisfactory 64%
frustrated 63%
sad 60%

In step S37, the words ['pleased', 'unhappy'] are assigned as new possible answers to the question and in step S38 the model is used to predict a subject next word among the new possible answers for prediction of the model with the subject sequence (input seed) being "or the work you do?". The model prediction (predicted subject next word) is "unhappy" with 0.575 probability. Therefore the possible answer corresponding to "unhappy", which is "dissatisfied", is selected/stored as the answer to the question under consideration.

The final answers generated using the Categorical answer prediction model are shown in the table in Figure 14 (last two rows).

Metric calculation

Step S17 is carried out. The data from the wellbeing index metric data is used to assign values to the answers and the values are combined. In this example a normalized summation of each answer by wellbeing topic is presented in a radar chart as shown in Figure 14.

There are several tools available to asses health/wellbeing as a metric In the case of determining a diagnosis, a health metric may be considered as having two general dimensions: Presence of disease and absence of disease. Furthermore, in other examples the health metric may be a score and the value of the score may be used to determine the diagnosis.

Methods may use questionnaires available for self-assessment, for example, the NHS Depression and Anxiety self-assessment quiz: https://www.nhs.uk/mental-health/self-help/guides-tools-and-activities/depression-anxiety-self-assessment-quiz/

Other self-assessment questionnaires are publicly available to diagnose other diseases, for example:

> https://www.psycom.net/quizzes,
> https://www.psycom.net/am-i-an-alcoholic-alcohol-use-disorder,
> https://www.psycom.net/quizzes#bipolar,
> https://www.psycom.net/quizzes#ocd,
> https://www.psycom.net/quizzes#anxiety,
> https://www.psycom.net/quizzes#schizophrenia,
> https://www.psycom.net/quizzes#eating,
> https://www.psycom.net/quizzes#borderline, and
> https://www.psycom.net/quizzes#addiction.

Worked example - diagnosing depression

A worked example will now be described in which a health metric is computed and a diagnosis is determined based on the health metric. A system implementing the method receives the following inputs

> 1. The questionnaire and metric computation rules (i.e. rules for computing the health metric based on answers). The metric computation also includes rules for determining a diagnosis based on the health metric.

Text data with a target patient's information. This is any type of text that contains information about the target patient such as Electronic Health Records, records of previous consultations, diary of the target patient, etc.

The questions in this example (i.e. the questionnaire) are from the medically reviewed article https://www.everydayhealth.com/depression/5-questions-doctors-ask-when-screening-for-depression.aspx. This includes five of the most common questions that healthcare professionals ask when trying to diagnose depression. For convenience in describing this example the questions input in this example are limited to these five questions.

Questionnaire input:

> 1 "In the past two weeks, how often have you felt down, depressed, or hopeless?"
> 2 "Have you had any thoughts of suicide?"
> 3 "How is your sleep?"
> 4 "How is your energy?"
> 5 "Do you prefer to stay at home rather than going out and doing new things?"

Questionnaire interpretation (metric rules)

Each question answer corresponds to a value that represents how positive/negative in nature an answer is. The rules may include rules prescribing values for answers. The value is any real value from 0 to 1 where 0 is extremely negative and 1 is extremely positive. The weight of each answer and the quantification is prescribed by the rules (which may be based on the article which is reviewed. The weight of the answer to each question is:

> Question 1: 80%
> Question 2: 80%
> Question 3: 50%
> Question 4: 50%
> Question 5: 50%

Input Data: EHR of target patient. For this example excerpts of the full EHR text found at https://mtsamples.com/site/pages/sample.asp?Type=97-Consult%20-%20History%20and%20Phy.&Sample=215-Psych%20Consult%20-%20Depression%20-%201 are used.

Information extraction based on Input Data

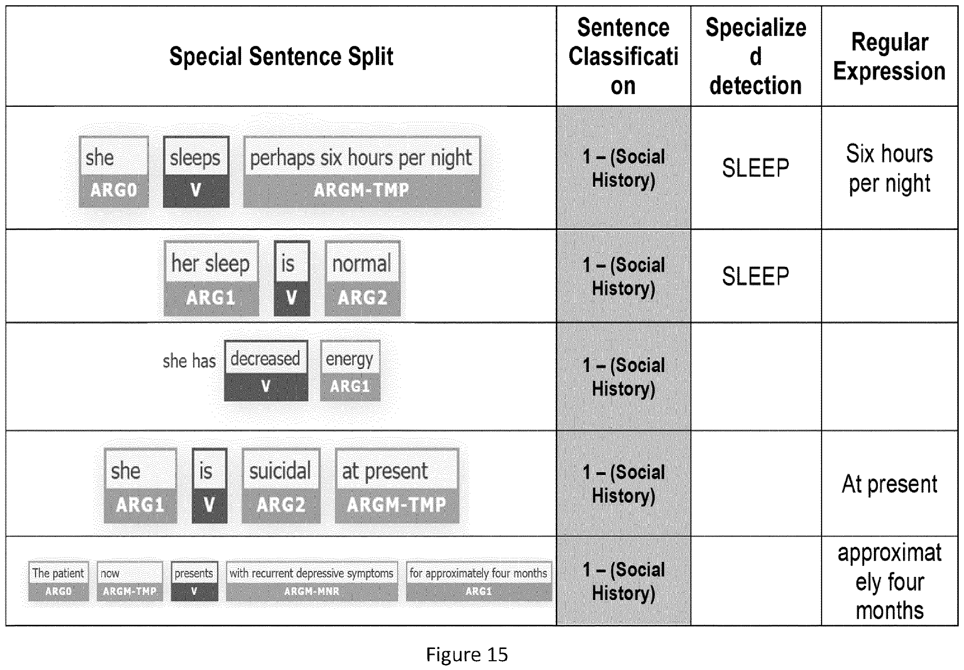

In this example only one EHR from the target patient is used, but in other examples information is extracted from more than one document in the input data. Social history information is extracted in the way described above with reference to Figure 2. The table shown in Figure 15 shows the extracted social history information. For convenience, the table only shows extracted information relevant to determining the health index and diagnosing depression.

Family history information extraction and medical NER extraction is also carried out but this is not shown or described, for convenience.

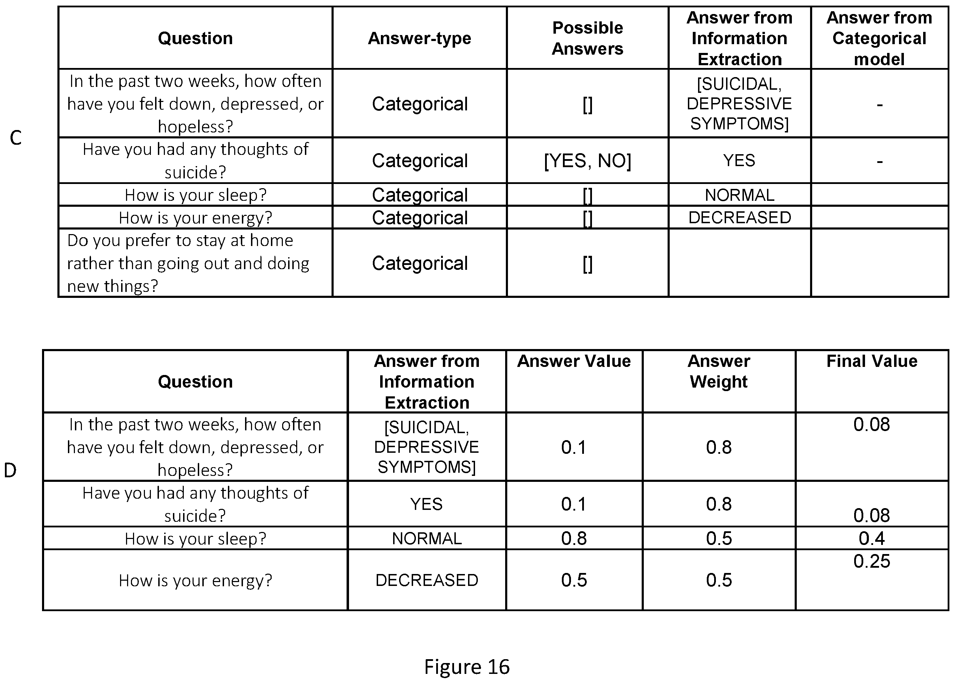

The Answer generation flow is followed to extract any possible answers from the extracted information. Table C in Figure 16 shows the extracted answers from the input data (EHR of target patient). Table D in Figure 16 shows the answers extracted from the extracted information. There is no field in table D labelled "Answer from numerical model" because all four of the questions considered here are categorical questions.

We can see that in the patients EHR there is not enough information to answer question 5. Whilst in some implementations a categorical answer may be predicted, for convenience in this example, the method proceeds based on the answers found.

For this example, a diagnosis of depression may be expressed as a probability of positive or negative diagnosis by evaluating a polarity of each answer. By doing so, a flexible value is added to categorical answers to open questions as opposed to a fixed value being added for categorical answers to fixed-set questions (e.g. yes/no type questions). This polarity of an answer may be determined using sentiment analysis. For example the Spacy textblob open library (https://spacy.io/universe/project/spacy-textblob) may be used for this task, and is used in the present example. According to this analysis, positive sentiments are closer to 1 and negative sentiments are closer to 0. Using this analysis, values are obtained for each answer. Determining the polarity as a binary value (0 or 1) may simplify the process. The value for each answer multiplied by the answer weight is computed and shown in the "final value" column of table D in Figure 16.

In some examples, a value for each answer may be obtained by referring to rules prescribing values for answers, for example in the form of a lookup table.

In this example, the average of the final values of answers is 0.2025. Since, based on the sentiment analysis, positive outcomes are closer to 1 and negative outcomes are closer to 0, the patient has 1 - 0.2025 = 0.7975 probability to be diagnosed with depression. This value may be compared to a threshold to determine a diagnosis. For example, the threshold may be prescribed in the rules input to a system implementing the method. In this example the threshold is taken to be 0.5. Since the value is above 0.5, a diagnosis of depression is determined. Another way to view the value of 0.7975 is that the target patient may be diagnosed with depression 79% or 80% of the time.

The value of 0.2025 or the value of 0.7975 may be considered to be the health metric (which value is the health metric may be determined by the rules for metric computation) and may be compared with a health metric threshold to determine a diagnosis of depression. Such a health metric threshold may be defined in the rules.

Worked example - diagnosing alcoholism

For this example, the self-assessment test from https://www.drinkaware.co.uk/tools/self-assessment is used to provide questions for the method. This is a nine-question test with multiple choice answers (i.e. fixed-set questions). For convenience in this example, four of the questions are considered.

Questions input for use in the method:

1. How often do you have a drink containing alcohol?
Possible answers:

    Never
    Monthly or less
    2-4 times a month
    2-3 times a week
    4 or more times per week

2. How often do you have 8 or more units on one occasion?
Possible answers:

    Never
    Less than monthly
    Monthly
    Weekly
    Daily or almost

3. How often during the last year have you failed to do what was normally expected from you because of your drinking?
Possible answers:

    Never
    Less than monthly
    Monthly
    Weekly
    Daily or almost

4. How often during the last year have you found that you were not able to stop drinking once you had started?
Possible answers:

    Never
    Less than monthly
    Monthly
    Weekly
    Daily or almost

The questionnaire interpretation may be prescribed by rules for computing the metric, including what values to assign answers to each question and weights for combining the values for the answers. In this example, rules prescribe that each answer yields a numeric value that represents how positive/negative in nature an answer is, and that the value assigned depends on the "position" of the answer (i.e. whether the first, second, third, fourth, or fifth answer is chosen). In particular, the rules prescribe the following values in this example for each question:

    Answer in position 1: 0
    Answer in position 2: 0.4

Answer in position 3: 0.6
Answer in position 4: 0.8
Answer in position 5: 1

The rules in this example prescribe that a health metric is computed by calculating a normalized sum of the values for the selected answers for the questions. It is noted that answers in the 5th position indicate high probabilities of a positive alcoholism diagnosis, whilst answers in position 1 indicate low probabilities of a positive alcoholism diagnosis.

For this example excerpts of the EHR text found at https://mtsamples.com/site/pages/sample.asp?Type=97-Consult%20-%20History%20and%20Phy.&Sample=215-Psych%20Consult%20-%20Depression%20-%201 are used as the input data related to the target patient.

In this example only one EHR from the target patient is used, but in other examples information is extracted from more than one document in the input data.

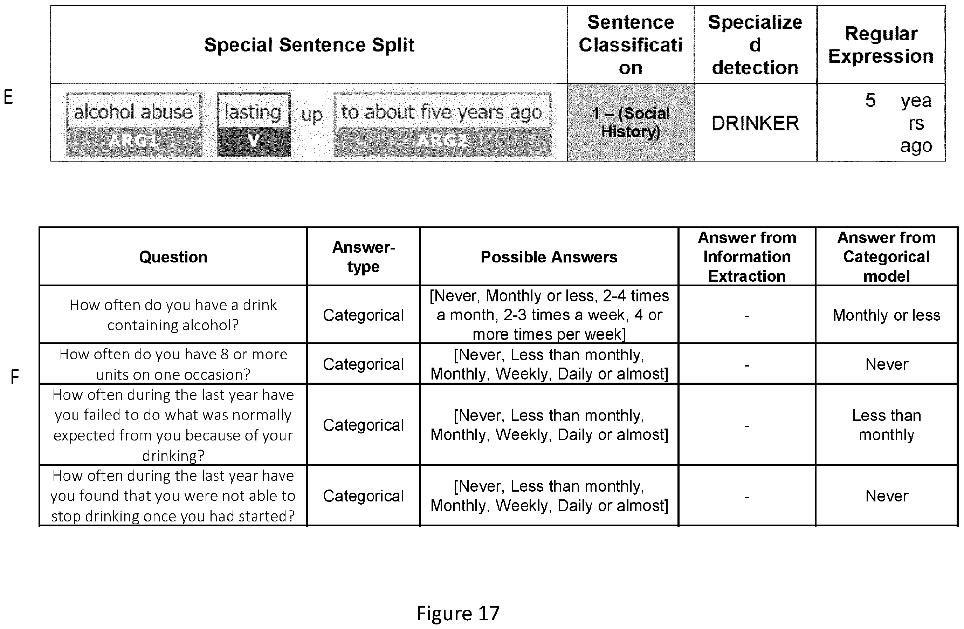

Social history information is extracted in the way described above with reference to Figure 2. The table E shown in Figure 17 shows some extracted social history information. For convenience, the table only shows extracted information relevant to determining the health index and diagnosing alcoholism.

Family history information extraction and medical NER extraction is also carried out but this is not shown or described, for convenience.

In the step S12 no answers to any question can be extracted from the extracted information and therefore the categorical answer prediction model is used to predict answers to the questions given that the questions are all categorical questions (step S12 -"no" to step S14 - "no" to step S16 which comprises using the categorical answer prediction model). Training data is generated based on the input data according to step S30 and a next-word prediction model is trained according to step S31. The next-word prediction model receives, for each question, a subject sequence of words and predicts a a plurality of preliminary subject next words based on the subject sequence of words, along with a probability of each preliminary subject next word being the correct next word. For the first question "How often do you have a drink containing alcohol?", the subject sequence of words (input to the trained next-word prediction model) is "How often do you have a drink containing alcohol?", and the preliminary subject next words (which are among the possible answers for the question under consideration) and their probabilities/scores are:

Never = 0.684
Monthly or less = 0.785
2-4 times a month = 0.021
2-3 times a week = 0.014
4 or more times per week = 0.024

The preliminary subject next word "monthly or less", having the highest score/probability, is selected as the predicted subject next word and selected/stored as the answer to the question under consideration (step S35). The trained next-word prediction model is used to make predictions over all questions in a similar way. The table F in Figure 17 shows the answers selected/stored for each question. There is no field in the table for the numerical answer prediction model because all of the questions considered are categorical questions.

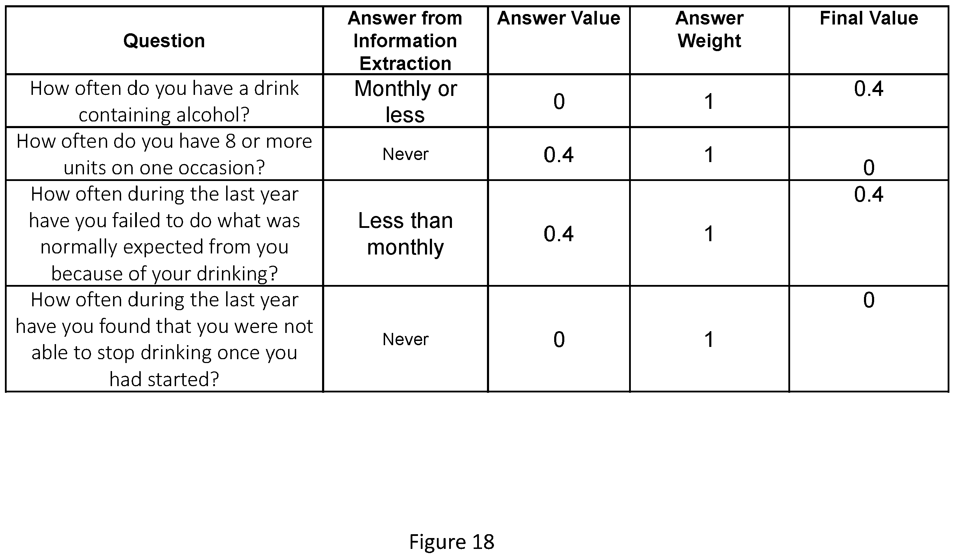

In step the health metric is computed according to the rules described above in this example. The table in Figure 18 shows the value assigned to each answer according to the rules. The average of the final values for the answers is 0.2. This health metric may be compared with a health metric threshold defined in the rules to determine a diagnosis of alcoholism (above threshold = alcoholic; equal to or less than threshold = not alcoholic). For example, the threshold may be 0.5, in which case the comparison yields a diagnosis that the target patient is not an alcoholic.

The health metric may be considered a probability for the target patient to be diagnosed with alcoholism. Given that this probability is 0.2, it may be considered that that the patient is not diagnosed with alcoholism.

The methods described above and systems implementing the methods may be for example integrated as a plugin inside existing current frameworks or integrated within a bigger clinical decision support system for healthcare applications.

Technical benefits of this invention include:

- Fully automated computation of health/wellbeing index metrics
- Fully automated extraction of Social history information
- Robust calculation of health/wellbeing metric even in the case of missing information (i.e. if input data relating to the target patient does not contain information to respond to a question, an approximate answer is generated/predicted)

In an example, an apparatus or system may be or comprise an automated metric generator of subjective wellbeing index metrics that processes unstructured textual data to generate answers to a wellbeing questionnaire and thus generate the wellbeing index metric. The system may comprise three modules that work alongside each other to generate answers for metric calculation:

Information Extraction (with Social history extraction)
Numerical Answer prediction model
Categorical Answer prediction model.

The social history extraction module may follow a flow/method with key algorithms modified to suit this task:

Special Sentence split to tokenize sentences in more modular manner instead of the usual punctuation tokenization of sentences;

Sentence classification to discard sentences that do not include social history information;

Specialized detection of entities in sentences to categorize based on the specific topics of social history; and

Regular expression to find numerical values and their units of measurement.

The Numerical Answer prediction model may follows a flow/method with key algorithmic procedures to find numerical answers by similarity between documents based on characteristic keywords from the question. The Categorical Answer prediction model may combine next-word learning architecture with word vectorization to alleviate the issue of questions requiring particular possible answers which are not in the vocabulary of a next-word prediction model. An Answer Manager engine may provide predicted answers in such cases where information for answering a question is missing from the input data.

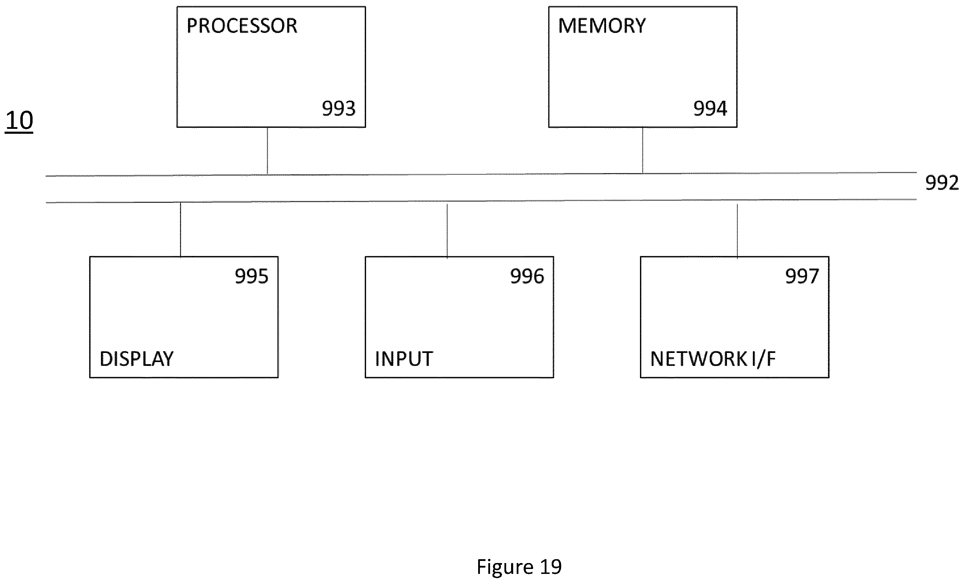

Figure 19 is a block diagram of an information processing apparatus 10 or a computing device 10, such as a data storage server, which embodies the present invention, and which may be used to implement some or all of the operations of a method embodying the present invention, and perform some or all of the tasks of apparatus of an embodiment. The computing device 10 may be used to implement any of the method steps described above, e.g. any of S10-S17 in Figure 1, S51-S58 in Figure 5, S20-S25 in Figure 6, and S30-S38 in Figure 7.

The computing device 10 comprises a processor 993 and memory 994. Optionally, the computing device also includes a network interface 997 for communication with other such computing devices, for example with other computing devices of invention embodiments. Optionally, the computing device also includes one or more input mechanisms such as keyboard and mouse 996, and a display unit such as one or more monitors 995. These elements may facilitate user interaction. The components are connectable to one another via a bus 992.

The memory 994 may include a computer readable medium, which term may refer to a single medium or multiple media (e.g., a centralized or distributed database and/or associated caches and servers) configured to carry computer-executable instructions. Computer-executable instructions may include, for example, instructions and data accessible by and causing a general purpose computer, special purpose computer, or special purpose processing device (e.g., one or more processors) to perform one or more functions or operations. For example, the computer-executable instructions may include those instructions for implementing a method disclosed herein, or any method steps disclosed herein, for example any of S10-S17 in Figure 1, S51-S58 in Figure 5, S20-S25 in Figure 6, and S30-S38 in Figure 7. Thus, the term "computer-readable storage medium" may also include any medium that is capable of storing, encoding or carrying a set of instructions for execution by the machine and that cause the machine to perform any one or more of the method steps of the present disclosure. The term "computer-readable storage medium" may accordingly be taken to include, but not be limited to, solid-state memories, optical media and magnetic media. By way of example, and not limitation, such computer-readable media may include non-transitory computer-readable storage media, including Random Access Memory (RAM), Read-Only Memory (ROM), Electrically Erasable Programmable Read-Only Memory (EEPROM), Compact Disc Read-Only Memory (CD-ROM) or other optical disk storage, magnetic disk storage or other magnetic storage devices, flash memory devices (e.g., solid state memory devices).

The processor 993 is configured to control the computing device and execute processing operations, for example executing computer program code stored in the memory 994 to implement any of the method steps described herein. The memory 994 stores data being read and written by the processor 993 and may store unstructured (medical history) data and/or unstructured (medical history) training data and/or any of the extracted family history information and/or extracted social history information and/or extracted medical (history) information and/or input data and/or other input data and/or metric computation data/rules, described above and/or programs for executing any of the method steps described above, e.g. any of S10-S17 in Figure 1, S51-S58 in Figure 5, S20-S25 in Figure 6, and S30-S38 in Figure 7, and/or any of the rule-based algorithms described above. As referred to herein, a processor may include one or more general-purpose processing devices such as a microprocessor, central processing unit, or the like. The processor may include a complex instruction set computing (CISC) microprocessor, reduced instruction set computing (RISC) microprocessor, very long instruction word (VLIW) microprocessor, or a processor implementing other instruction sets or processors implementing a combination of instruction sets. The processor may also include one or more special-purpose processing devices such as an application specific integrated circuit (ASIC), a field programmable gate array (FPGA), a digital signal processor (DSP), network processor, or the like. In one or more embodiments, a processor is configured to execute instructions for performing the operations and operations discussed herein. The processor 993 may be considered to comprise any of the modules described above. Any operations described as being implemented by a module may be implemented as a method by a computer and e.g. by the processor 993.

The display unit 995 may display a representation of data stored by the computing device, such as a representation of a health index or a diagnosis or a recommendation and/or GUI windows and/or interactive representations enabling a user to interact with the apparatus 10 by e.g. drag and drop or selection interaction, and/or any other output described above, and may also display a cursor and dialog boxes and screens enabling interaction between a user and the programs and data stored on the computing device. The input mechanisms 996 may enable a user to input data and instructions to the computing device, such as enabling a user to input any user input described above.

The network interface (network I/F) 997 may be connected to a network, such as the Internet, and is connectable to other such computing devices via the network. The network I/F 997 may control data input/output from/to other apparatus via the network. Other peripheral devices such as microphone, speakers, printer, power supply unit, fan, case, scanner, trackerball etc may be included in the computing device.

Methods embodying the present invention may be carried out on a computing device/apparatus 10 such as that illustrated in Figure 19. Such a computing device need not have every component illustrated in Figure 19, and may be composed of a subset of those components. For example, the apparatus 10 may comprise the processor 993 and the memory 994 connected to the processor 993. Or the apparatus 10 may comprise the processor 993, the memory 994 connected to the processor 993, and the display 995. A method embodying the present invention may be carried out by a single computing device in communication with one or more data storage servers via a network. The computing device may be a data storage itself storing at least a portion of the data.

A method embodying the present invention may be carried out by a plurality of computing devices operating in cooperation with one another. One or more of the plurality of computing devices may be a data storage server storing at least a portion of the data.

The invention may be implemented in digital electronic circuitry, or in computer hardware, firmware, software, or in combinations of them. The invention may be implemented as a computer program or computer program product, i.e., a computer program tangibly embodied in a non-transitory information carrier, e.g., in a machine-readable storage device, or in a propagated signal, for execution by, or to control the operation of, one or more hardware modules.

A computer program may be in the form of a stand-alone program, a computer program portion or more than one computer program and may be written in any form of programming language, including compiled or interpreted languages, and it may be deployed in any form, including as a stand-alone program or as a module, component, subroutine, or other unit suitable for use in a data processing environment. A computer program may be deployed to be executed on one module or on multiple modules at one site or distributed across multiple sites and interconnected by a communication network.

Method steps of the invention may be performed by one or more programmable processors executing a computer program to perform functions of the invention by operating on input data and generating output. Apparatus of the invention may be implemented as programmed hardware or as special purpose logic circuitry, including e.g., an FPGA (field programmable gate array) or an ASIC (application-specific integrated circuit).

Processors suitable for the execution of a computer program include, by way of example, both general and special purpose microprocessors, and any one or more processors of any kind of digital computer. Generally, a processor will receive instructions and data from a read-only memory or a random access memory or both. The essential elements of a computer are a processor for executing instructions coupled to one or more memory devices for storing instructions and data.

The above-described embodiments of the present invention may advantageously be used independently of any other of the embodiments or in any feasible combination with one or more others of the embodiments.

## Claims

1. A computer-implemented method comprising:

   extracting information from unstructured data relating to a target patient;
   categorizing each of a plurality of questions as a numerical question requiring a numerical answer or a categorical question requiring a categorical answer, based on a set of question keywords and corresponding question keyword categorizations; and
   for each question, when the extracted information includes patient information corresponding to an answer to the question, selecting that patient information as the answer, and when the extracted information does not include patient information corresponding to the answer, predicting the answer,
   wherein, for each question

      when the question is a numerical question requiring a numerical answer, predicting the numerical answer comprises:

         extracting characteristic keywords from the question;
         computing a relevance measure of at least one of the characteristic keywords in a document from the unstructured data relating to the target patient;
         computing a relevance measure of the at least one characteristic keyword in each of a plurality of documents relating to other patients;
         identifying one of the plurality of documents relating to the other patients in which the relevance measure of the at least one characteristic keyword is most similar to the relevance measure of the at least one characteristic keyword, respectively, in the document relating to the target patient;
extracting other patient information corresponding to the numerical answer from the identified document; and
selecting the extracted other patient information as the numerical answer,

and when the question is a categorical question requiring a categorical answer, predicting the categorical answer comprises:

using a machine-learning prediction model trained to predict a next word in a sequence of words from the unstructured data relating to the target patient given a plurality of words preceding the next word in the sequence, predicting a subject next word in a subject sequence of words comprising a plurality of words extracted from the question; and
selecting a categorical answer based on the predicted subject next word,

wherein the computer-implemented method further comprises computing, using the selected answers and using rules, a health metric.

2. The computer-implemented method as claimed in claim 1, wherein extracting the information from the unstructured data relating to the target patient comprises:

parsing at least one sentence in the unstructured data to generate at least one constituent sentence; and
searching for topic keywords in the at least one constituent sentence and categorizing the at least one constituent sentence as corresponding to at least one of a plurality of topics based on any detected topic keywords.

3. The computer-implemented method as claimed in claim 1 or 2, wherein categorizing each of a plurality of questions comprises, for each question, searching for the question keywords in the question and, when a said question keyword is identified in the question, assigning the question keyword categorization corresponding to the question keyword.

4. The computer-implemented method as claimed in any of claims 1 to 3, further comprising classifying at least one question as a fixed-set question requiring an answer from a set of possible answers in the question or an open question requiring an open answer.

5. The computer-implemented method as claimed in claim 4, wherein classifying at least one question comprises identifying a noun that appears first in the question as an initial noun, and, if an adjective-chunk directly follows the initial noun, classifying the question as a fixed-set question.

6. The computer-implemented method as claimed in any of the preceding claims, wherein extracting characteristic keywords from a said numerical question comprises cleaning the question using stemming and stop-words removal and extracting the remaining words as characteristic keywords.

7. The computer-implemented method as claimed in any of the preceding claims, wherein each relevance measure is the term frequency-inverse document frequency, TF-IDF, of the characteristic keyword.

8. The computer-implemented method as claimed in any of the preceding claims, further comprising, before using the prediction model, training the prediction model by:

assembling training data by extracting sequences of words from the unstructured data and/or from the constituent sentences in the extracted information, removing the last word of each sequence to generate training sequences of words; and
training the prediction model to predict the next word in each training sequence of words, using for each training sequence of words the removed last word as ground-truth data.

9. The computer-implemented method as claimed in claim 8, wherein the subject sequence of words comprises the last n words of the question, n being an integer more than 1.

10. The computer-implemented method as claimed in any of the preceding claims, wherein, when the question is an open question and a categorical question, selecting the categorical answer based on the predicted subject next word comprises:
selecting the predicted subject next word as the categorical answer.

11. The computer-implemented method as claimed in any of the preceding claims, wherein computing the health metric comprises adding a metric score to a health metric for each of the plurality of questions.

**12.** The computer-implemented method as claimed in claim 11, wherein the rules define, for each categorical question, the metric score to be added to the health metric for each possible answer to the question.

**13.** The computer-implemented method as claimed in any of the preceding claims, further comprising determining and outputting a diagnosis according to the health metric.

**14.** A computer program which, when run on a computer, causes the computer to carry out a method comprising:

> extracting information from unstructured data relating to a target patient;
> categorizing each of a plurality of questions as a numerical question requiring a numerical answer or a categorical question requiring a categorical answer, based on a set of question keywords and corresponding question keyword categorizations; and
> for each question, when the extracted information includes patient information corresponding to an answer to the question, selecting that patient information as the answer, and when the extracted information does not include patient information corresponding to the answer, predicting the answer,
> wherein, for each question

>> when the question is a numerical question requiring a numerical answer, predicting the numerical answer comprises:

>>> extracting characteristic keywords from the question;
>>> computing a relevance measure of at least one of the characteristic keywords in a document from the unstructured data relating to the target patient;
>>> computing a relevance measure of the at least one characteristic keyword in each of a plurality of documents relating to other patients;
>>> identifying one of the plurality of documents relating to the other patients in which the relevance measure of the at least one characteristic keyword is most similar to the relevance measure of the at least one characteristic keyword, respectively, in the document relating to the target patient;
>>> extracting other patient information corresponding to the numerical answer from the identified document; and
>>> selecting the extracted other patient information as the numerical answer,

and when the question is a categorical question requiring a categorical answer, predicting the categorical answer comprises:

> using a machine-learning prediction model trained to predict a next word in a sequence of words from the unstructured data relating to the target patient given a plurality of words preceding the next word in the sequence, predicting a subject next word in a subject sequence of words comprising a plurality of words extracted from the question; and
> selecting a categorical answer based on the predicted subject next word,

wherein the method further comprises computing, using the selected answers and using rules, a health metric.

**15.** An information processing apparatus comprising a memory and a processor connected to the memory, wherein the processor is configured to:

> extract information from unstructured data relating to a target patient;
> categorize each of a plurality of questions as a numerical question requiring a numerical answer or a categorical question requiring a categorical answer, based on a set of question keywords and corresponding question keyword categorizations; and
> for each question, when the extracted information includes patient information corresponding to an answer to the question, select that patient information as the answer, and when the extracted information does not include patient information corresponding to the answer, predict the answer,
> wherein, for each question

>> when the question is a numerical question requiring a numerical answer, predicting the numerical answer comprises:

>>> extracting characteristic keywords from the question;
>>> computing a relevance measure of at least one of the characteristic keywords in a document from the unstructured data relating to the target patient;
>>> computing a relevance measure of the at least one characteristic keyword in each of a plurality of documents relating to other patients;
identifying one of the plurality of documents relating to the other patients in which the relevance measure of the at least one characteristic keyword is most similar to the relevance measure of the at least one characteristic keyword, respectively, in the document relating to the target patient;
extracting other patient information corresponding to the numerical answer from the identified document; and
selecting the extracted other patient information as the numerical answer,

and when the question is a categorical question requiring a categorical answer, predicting the categorical answer comprises:

using a machine-learning prediction model trained to predict a next word in a sequence of words from the unstructured data relating to the target patient given a plurality of words preceding the next word in the sequence, predicting a subject next word in a subject sequence of words comprising a plurality of words extracted from the question; and
selecting a categorical answer based on the predicted subject next word,

wherein the processor is further configured to compute, using the selected answers and using rules, a health metric.

**Amended claims in accordance with Rule 137(2) EPC.**

1. A computer-implemented method comprising:

extracting information from unstructured data relating to a target patient;
categorizing (S10) each of a plurality of questions as a numerical question requiring a numerical answer or a categorical question requiring a categorical answer, by searching for question keywords in the question and categorizing the question based on a set of question keywords and corresponding question keyword categorizations; and
for each question, when the extracted information includes patient information corresponding to an answer to the question, selecting (S13) that patient information as the answer, and when the extracted information does not include patient information corresponding to the answer, predicting the answer,
wherein, for each question

when the question is a numerical question requiring a numerical answer, predicting (S15) the numerical answer comprises:

extracting characteristic keywords from the question;
computing (S23) a relevance measure of at least one of the characteristic keywords in a document from the unstructured data relating to the target patient;
computing (S24) a relevance measure of the at least one characteristic keyword in each of a plurality of documents relating to other patients;
identifying (S25) one of the plurality of documents relating to the other patients in which the relevance measure of the at least one characteristic keyword is most similar to the relevance measure of the at least one characteristic keyword, respectively, in the document relating to the target patient;
extracting other patient information corresponding to the numerical answer from the identified document; and
selecting the extracted other patient information as the numerical answer,

and when the question is a categorical question requiring a categorical answer, predicting (S16) the categorical answer comprises:

using a machine-learning prediction model trained to predict a next word in a sequence of words from the unstructured data relating to the target patient given a plurality of words preceding the next word in the sequence, predicting a subject next word in a subject sequence of words comprising a plurality of words extracted from the question; and
selecting a categorical answer based on the predicted subject next word,

wherein the computer-implemented method further comprises computing (S17), using the selected answers and using rules, a health metric, wherein computing (S23) the relevance measure of the at least one of the characteristic keywords in the document from the unstructured data relating to the target patient comprises computing the term frequency-inverse document frequency, TF-IDF, of the characteristic keyword by: calculating a term frequency of the characteristic keyword in the document; calculating the inverse document frequency of the characteristic keyword in all documents in the unstructured data relating to the target patient; and computing the product of the term frequency and the inverse document frequency,

and wherein computing (S24) the relevance measure of the at least one characteristic keyword in each of the plurality of documents relating to other patients comprises, for each document, computing the TF-IDF of the characteristic keyword by: calculating a term frequency of the characteristic keyword in the document; calculating the inverse document frequency of the characteristic keyword in all documents relating to the other patients; and computing the product of the term frequency and the inverse document frequency.

2. The computer-implemented method as claimed in claim 1, wherein extracting the information from the unstructured data relating to the target patient comprises:

parsing at least one sentence in the unstructured data to generate at least one constituent sentence; and
searching for topic keywords in the at least one constituent sentence and categorizing the at least one constituent sentence as corresponding to at least one of a plurality of topics based on any detected topic keywords.

3. The computer-implemented method as claimed in claim 1 or 2, wherein categorizing (S10) each of a plurality of questions comprises, for each question, searching for the question keywords in the question and, when a said question keyword is identified in the question, assigning the question keyword categorization corresponding to the question keyword.

4. The computer-implemented method as claimed in any of claims 1 to 3, further comprising classifying (S11) at least one question as a fixed-set question requiring an answer from a set of possible answers in the question or an open question requiring an open answer.

5. The computer-implemented method as claimed in claim 4, wherein classifying (S11) at least one question comprises identifying a noun that appears first in the question as an initial noun, and, if an adjective-chunk directly follows the initial noun, classifying the question as a fixed-set question.

6. The computer-implemented method as claimed in any of the preceding claims, wherein extracting characteristic keywords from a said numerical question comprises cleaning (S20) the question using stemming and stop-words removal and extracting the remaining words as characteristic keywords.

7. The computer-implemented method as claimed in any of the preceding claims, further comprising, before using the prediction model, training (S31) the prediction model by:

assembling (S30) training data by extracting sequences of words from the unstructured data and/or from the constituent sentences in the extracted information, removing the last word of each sequence to generate training sequences of words; and
training the prediction model to predict the next word in each training sequence of words, using for each training sequence of words the removed last word as ground-truth data.

8. The computer-implemented method as claimed in claim 7, wherein the subject sequence of words comprises the last n words of the question, n being an integer more than 1.

9. The computer-implemented method as claimed in any of the preceding claims, wherein, when the question is an open question and a categorical question, selecting the categorical answer based on the predicted subject next word comprises:
selecting the predicted subject next word as the categorical answer.

10. The computer-implemented method as claimed in any of the preceding claims, wherein computing (S17) the health metric comprises adding a metric score to a health metric for each of the plurality of questions.

11. The computer-implemented method as claimed in claim 10, wherein the rules define, for each categorical question, the metric score to be added to the health metric for each possible answer to the question.

12. The computer-implemented method as claimed in any of the preceding claims, further comprising determining a diagnosis by comparing the health metric with a threshold health metric and outputting the diagnosis.

13. A computer program which, when run on a computer, causes the computer to carry out a method comprising:

extracting information from unstructured data relating to a target patient;

categorizing (S10) each of a plurality of questions as a numerical question requiring a numerical answer or a categorical question requiring a categorical answer, by searching for question keywords in the question and categorizing the question based on a set of question keywords and corresponding question keyword categorizations; and

for each question, when the extracted information includes patient information corresponding to an answer to the question, selecting (S13) that patient information as the answer, and when the extracted information does not include patient information corresponding to the answer, predicting the answer,

wherein, for each question

when the question is a numerical question requiring a numerical answer, predicting (S15) the numerical answer comprises:

extracting characteristic keywords from the question;

computing (S23) a relevance measure of at least one of the characteristic keywords in a document from the unstructured data relating to the target patient;

computing (S24) a relevance measure of the at least one characteristic keyword in each of a plurality of documents relating to other patients;

identifying (S25) one of the plurality of documents relating to the other patients in which the relevance measure of the at least one characteristic keyword is most similar to the relevance measure of the at least one characteristic keyword, respectively, in the document relating to the target patient;

extracting other patient information corresponding to the numerical answer from the identified document; and

selecting the extracted other patient information as the numerical answer,

and when the question is a categorical question requiring a categorical answer, predicting (S16) the categorical answer comprises:

using a machine-learning prediction model trained to predict a next word in a sequence of words from the unstructured data relating to the target patient given a plurality of words preceding the next word in the sequence, predicting a subject next word in a subject sequence of words comprising a plurality of words extracted from the question; and

selecting a categorical answer based on the predicted subject next word,

wherein the method further comprises computing (S17), using the selected answers and using rules, a health metric,

wherein computing (S23) the relevance measure of the at least one of the characteristic keywords in the document from the unstructured data relating to the target patient comprises computing the term frequency-inverse document frequency, TF-IDF, of the characteristic keyword by: calculating a term frequency of the characteristic keyword in the document; calculating the inverse document frequency of the characteristic keyword in all documents in the unstructured data relating to the target patient; and computing the product of the term frequency and the inverse document frequency,

and wherein computing (S24) the relevance measure of the at least one characteristic keyword in each of the plurality of documents relating to other patients comprises, for each document, computing the TF-IDF of the characteristic keyword by: calculating a term frequency of the characteristic keyword in the document; calculating the inverse document frequency of the characteristic keyword in all documents relating to the other patients; and computing the product of the term frequency and the inverse document frequency.

14. An information processing apparatus (10) comprising a memory (994) and a processor (993) connected to the memory (994), wherein the processor (993) is configured to:

extract information from unstructured data relating to a target patient;

categorize each of a plurality of questions as a numerical question requiring a numerical answer or a categorical question requiring a categorical answer, by searching for question keywords in the question and categorizing the question based on a set of question keywords and corresponding question keyword categorizations; and

for each question, when the extracted information includes patient information corresponding to an answer to the question, select that patient information as the answer, and when the extracted information does not include patient information corresponding to the answer, predict the answer,

wherein, for each question

when the question is a numerical question requiring a numerical answer, predicting the numerical answer comprises:

> extracting characteristic keywords from the question;
> computing a relevance measure of at least one of the characteristic keywords in a document from the unstructured data relating to the target patient;
> computing a relevance measure of the at least one characteristic keyword in each of a plurality of documents relating to other patients;
> identifying one of the plurality of documents relating to the other patients in which the relevance measure of the at least one characteristic keyword is most similar to the relevance measure of the at least one characteristic keyword, respectively, in the document relating to the target patient;
> extracting other patient information corresponding to the numerical answer from the identified document; and
> selecting the extracted other patient information as the numerical answer,

and when the question is a categorical question requiring a categorical answer, predicting the categorical answer comprises:

> using a machine-learning prediction model trained to predict a next word in a sequence of words from the unstructured data relating to the target patient given a plurality of words preceding the next word in the sequence, predicting a subject next word in a subject sequence of words comprising a plurality of words extracted from the question; and
> selecting a categorical answer based on the predicted subject next word,

wherein the processor is further configured to compute, using the selected answers and using rules, a health metric,
wherein computing the relevance measure of the at least one of the characteristic keywords in the document from the unstructured data relating to the target patient comprises computing the term frequency-inverse document frequency, TF-IDF, of the characteristic keyword by: calculating a term frequency of the characteristic keyword in the document; calculating the inverse document frequency of the characteristic keyword in all documents in the unstructured data relating to the target patient; and computing the product of the term frequency and the inverse document frequency,

and wherein computing the relevance measure of the at least one characteristic keyword in each of the plurality of documents relating to other patients comprises, for each document, computing the TF-IDF of the characteristic keyword by: calculating a term frequency of the characteristic keyword in the document; calculating the inverse document frequency of the characteristic keyword in all documents relating to the other patients; and computing the product of the term frequency and the inverse document frequency.

Figure 1

Figure 2

Figure 3

Figure 4

| S51 | Process Tokenization, Noun Detection, POS tagging and Syntax Tree Dependencies |
|-----|------------------------------------------------------------------------------|

| S52 | Extract family member roles |
|-----|------------------------------|

| S53 | Extract family side associated to each family role |
|-----|-----------------------------------------------------|

| S54 | Extract status associated to each family role |
|-----|------------------------------------------------|

| S55 | Extract observations associated to each family role |
|-----|------------------------------------------------------|

| S56 | Extract modality of observations (pos, neg) associated to each family role |
|-----|----------------------------------------------------------------------------|

| S57 | Build annotated dataset in JSON from extracted entities and relations |
|-----|-----------------------------------------------------------------------|

| S58 | Transform annotated dataset to BIO format |
|-----|--------------------------------------------|

Figure 5

Figure 6

Figure 7

EP 4 270 403 A1

```
Model: "sequential"

Layer (type)                Output Shape              Param #
=================================================================
embedding (Embedding)       (None, 1, 10)             26170

lstm (LSTM)                 (None, 1, 1000)           4044000

lstm_1 (LSTM)               (None, 1000)              8004000

dense (Dense)               (None, 1000)              1001000

dense_1 (Dense)             (None, 2617)              2619617
=================================================================
Total params: 15,694,787
Trainable params: 15,694,787
Non-trainable params: 0
```

Figure 8

| Question | Answer-type | Answer-value-type | Possible Answers |
|---|---|---|---|
| Do you smoke? | Categorical | Open | [] |
| In the last seven days, on how many days did you exercise for 30 minutes or more? | Numerical | Open | [] |
| Did you eat healthy all day yesterday? | Categorical | Open | [] |
| Are you satisfied or dissatisfied with your job or the work you do? | Categorical | Fixed Set | ['satisfied', 'dissatisfied'] |

Figure 9

| Special Sentence Split | | | | Sentence Classification | Specialized detection | Regular Expression |
|---|---|---|---|---|---|---|
| The patient `ARG1` | is `V` | married `ARG2` | | 0 – (Not Social History) | - | - |
| She `ARG1` | is | employed `V` | as an office manager `ARG2` | 0 – (Not Social History) | - | - |
| She `ARG0` does | smoke `V` | cigarettes `ARG1` , | one pack `ARGM-ADV` | 1 – (Social History) | SMOKER | 1 pack per day |
| She `ARG0` | consumes `V` | alcohol 3 to 5 drinks daily `ARG1` | | 1 – (Social History) | DRINKER | 3 to 5 drinks daily |
| She `ARG0` | uses `V` | no illicit drugs `ARG1` | | 1 – (Social History) | DRUG | - |
| She `ARG0` | exercises `V` | monthly mainly walking and low impact aerobics `ARG1` | | 1 – (Social History) | EXERCISE | - |
| She exercises `ARG0` | monthly | mainly `ARGM-ADV` | walking `V` | 1 – (Social History) | EXERCISE | - |
| She `ARG0` | also `ARGM-ADV` | likes `V` | to play softball `ARG1` | 1 – (Social History) | EXERCISE | - |
| She `ARG0` | also likes to | play `V` | softball `ARG1` | 1 – (Social History) | EXERCISE | - |

Figure 10

| | text | label_ | start | end | start_char | end_char |
|---|---|---|---|---|---|---|
| 0 | depression | DISEASE | 6 | 7 | 38 | 48 |
| 1 | reflux disease | DISEASE | 8 | 10 | 53 | 67 |
| 2 | drug allergies | DISEASE | 43 | 45 | 222 | 236 |
| 3 | alcohol | CHEMICAL | 80 | 81 | 396 | 403 |
| 4 | nausea | DISEASE | 120 | 121 | 609 | 615 |
| 5 | anxiety | DISEASE | 124 | 125 | 627 | 634 |
| 6 | depression | DISEASE | 126 | 127 | 639 | 649 |

Figure 11

**TF-IDF table from patient data**

| doc_id | keyword | TF-IDF |
|--------|---------|--------|
| EHR | days | 0.01 |
| EHR | exercise | 0.9 |
| EHR | many | 0.8 |
| EHR | 30 minutes | 0.12 |

A

**TF-IDF table from external data**

| doc_id | keyword | TF-IDF |
|--------|---------|--------|
| 1 | days | 0.5 |
| 1 | exercise | 0.75 |
| 1 | many | 0.56 |
| 1 | 30 minutes | 0.11 |
| 2 | days | 0.04 |
| 2 | exercise | 0.87 |
| 2 | many | 0.75 |
| 2 | 30 minutes | 0.14 |
| 3 | days | 0.8 |
| 3 | exercise | 0.9 |
| 3 | many | 0.7 |
| 3 | 30 minutes | 0.7 |

B

Figure 12

EP 4 270 403 A1

| | Sequence | Prediction |
|---|---|---|
| 0 | [She, does, smoke, cigarettes,, one] | pack |
| 1 | [does, smoke, cigarettes,, one, pack] | per |
| 2 | [smoke, cigarettes,, one, pack, per] | day |
| 3 | [cigarettes,, one, pack, per, day] | for |
| 4 | [one, pack, per, day, for] | the |
| 5 | [pack, per, day, for, the] | last |
| 6 | [per, day, for, the, last] | 20 |
| 7 | [day, for, the, last, 20] | years. |
| 8 | [for, the, last, 20, years.] | She |
| 9 | [the, last, 20, years., She] | consumes |
| 10 | [last, 20, years., She, consumes] | alcohol |
| 11 | [20, years., She, consumes, alcohol] | 3 |
| 12 | [years., She, consumes, alcohol, 3] | to |
| 13 | [She, consumes, alcohol, 3, to] | 5 |
| 14 | [consumes, alcohol, 3, to, 5] | drinks |
| 15 | [alcohol, 3, to, 5, drinks] | daily. |
| 16 | [3, to, 5, drinks, daily.] | She |
| 17 | [to, 5, drinks, daily., She] | uses |
| 18 | [5, drinks, daily., She, uses] | no |
| 19 | [drinks, daily., She, uses, no] | illicit |
| 20 | [daily., She, uses, no, illicit] | drugs. |

Figure 13

| Question | Answer-type | Possible Answers | Answer from Information Extraction | Answer from numerical model | Answer from Categorical model |
|---|---|---|---|---|---|
| Do you smoke? | Categorical | [] | Yes | - | - |
| In the last seven days, on how many days did you exercise for 30 minutes or more? | Numerical | [] | - | 1 | - |
| Did you eat healthy all day yesterday? | Categorical | [] | - | - | no |
| Are you satisfied or dissatisfied with your job or the work you do? | Categorical | ['satisfied', 'dissatisfied'] | - | - | dissatisfied |

Figure 14

| Special Sentence Split | Sentence Classification | Specialized detection | Regular Expression |
|---|---|---|---|
| she [ARG0] sleeps [V] perhaps six hours per night [ARGM-TMP] | 1 – (Social History) | SLEEP | Six hours per night |
| her sleep [ARG1] is [V] normal [ARG2] | 1 – (Social History) | SLEEP | |
| she has decreased [V] energy [ARG1] | 1 – (Social History) | | |
| she [ARG1] is [V] suicidal [ARG2] at present [ARGM-TMP] | 1 – (Social History) | | At present |
| The patient [ARG0] now [ARGM-TMP] presents [V] with recurrent depressive symptoms [ARGM-MNR] for approximately four months [ARG1] | 1 – (Social History) | | approximately four months |

Figure 15

EP 4 270 403 A1

C

| Question | Answer-type | Possible Answers | Answer from Information Extraction | Answer from Categorical model |
|---|---|---|---|---|
| In the past two weeks, how often have you felt down, depressed, or hopeless? | Categorical | [] | [SUICIDAL, DEPRESSIVE SYMPTOMS] | - |
| Have you had any thoughts of suicide? | Categorical | [YES, NO] | YES | - |
| How is your sleep? | Categorical | [] | NORMAL | |
| How is your energy? | Categorical | [] | DECREASED | |
| Do you prefer to stay at home rather than going out and doing new things? | Categorical | [] | | |

D

| Question | Answer from Information Extraction | Answer Value | Answer Weight | Final Value |
|---|---|---|---|---|
| In the past two weeks, how often have you felt down, depressed, or hopeless? | [SUICIDAL, DEPRESSIVE SYMPTOMS] | 0.1 | 0.8 | 0.08 |
| Have you had any thoughts of suicide? | YES | 0.1 | 0.8 | 0.08 |
| How is your sleep? | NORMAL | 0.8 | 0.5 | 0.4 |
| How is your energy? | DECREASED | 0.5 | 0.5 | 0.25 |

Figure 16

| Special Sentence Split | | | | Sentence Classification | Specialized detection | Regular Expression |
|---|---|---|---|---|---|---|
| alcohol abuse ARG1 | lasting V | up | to about five years ago ARG2 | 1 – (Social History) | DRINKER | 5 years ago |

| Question | Answer-type | Possible Answers | Answer from Information Extraction | Answer from Categorical model |
|---|---|---|---|---|
| How often do you have a drink containing alcohol? | Categorical | [Never, Monthly or less, 2-4 times a month, 2-3 times a week, 4 or more times per week] | - | Monthly or less |
| How often do you have 8 or more units on one occasion? | Categorical | [Never, Less than monthly, Monthly, Weekly, Daily or almost] | - | Never |
| How often during the last year have you failed to do what was normally expected from you because of your drinking? | Categorical | [Never, Less than monthly, Monthly, Weekly, Daily or almost] | - | Less than monthly |
| How often during the last year have you found that you were not able to stop drinking once you had started? | Categorical | [Never, Less than monthly, Monthly, Weekly, Daily or almost] | - | Never |

Figure 17

| Question | Answer from Information Extraction | Answer Value | Answer Weight | Final Value |
|---|---|---|---|---|
| How often do you have a drink containing alcohol? | Monthly or less | 0 | 1 | 0.4 |
| How often do you have 8 or more units on one occasion? | Never | 0.4 | 1 | 0 |
| How often during the last year have you failed to do what was normally expected from you because of your drinking? | Less than monthly | 0.4 | 1 | 0.4 |
| How often during the last year have you found that you were not able to stop drinking once you had started? | Never | 0 | 1 | 0 |

Figure 18

EP 4 270 403 A1

Figure 19

EP 4 270 403 A1

Europäisches
Patentamt

European
Patent Office

Office européen
des brevets

# EUROPEAN SEARCH REPORT

**Application Number**

EP 22 38 2393

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2020/176098 A1 (LUCAS MICHAEL [US] ET AL) 4 June 2020 (2020-06-04) <br> * claims 1-12,27 * <br> * paragraph [0002] * <br> * paragraph [0006] * <br> * paragraph [0024] - paragraph [0027] * <br> * paragraph [0055] * <br> * paragraph [0063] - paragraph [0094] * <br> * paragraph [0105] * <br> * paragraph [0117] - paragraph [0155] * <br> * paragraph [0157] - paragraph [0160] * <br> * paragraph [0162] * <br> * paragraph [0169] * <br> * paragraph [0182] * <br> * paragraph [0238] * <br> * paragraph [0250] * <br> * paragraph [0252] - paragraph [0253] * <br> ----- | 1-15 | INV. <br> G16H10/20 <br> G16H50/20 <br> G16H50/30 <br> G06F16/35 <br> G06F40/30 <br><br> ADD. <br> G06K9/62 |
| X | US 9 984 772 B2 (SIEMENS HEALTHCARE GMBH [DE]) 29 May 2018 (2018-05-29) <br> * claims 1-20 * <br> * paragraph [0005] - paragraph [0007] * <br> * paragraph [0019] - paragraph [0025] * <br> ----- | 1-15 | |
| A | US 10 796 102 B2 (ORACLE INT CORP [US]) 6 October 2020 (2020-10-06) <br> * paragraph [0203] * <br> * paragraph [0305] * <br> ----- | 1-15 | TECHNICAL FIELDS SEARCHED (IPC) <br><br> G16H <br> G06F <br> G06K |
| A | Anonymous: "IntelliSense in Visual Studio Code", <br><br> , <br> 20 April 2022 (2022-04-20), XP055969509, <br> Retrieved from the Internet: <br> URL:https://web.archive.org/web/2022042010 5848/https://code.visualstudio.com/docs/ed itor/intellisense <br> [retrieved on 2022-10-10] <br> * the whole document * <br> ----- | 1-15 | |

The present search report has been drawn up for all claims

| Place of search | Date of completion of the search | Examiner |
|---|---|---|
| Munich | 11 October 2022 | Martínez Cebollada |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 22 38 2393

This annex lists the patent family members relating to the patent documents cited in the above-mentioned European search report.
The members are as contained in the European Patent Office EDP file on
The European Patent Office is in no way liable for these particulars which are merely given for the purpose of information.

11-10-2022

| Patent document cited in search report | | Publication date | Patent family member(s) | | Publication date |
|---|---|---|---|---|---|
| US 2020176098 | A1 | 04-06-2020 | AU | 2019392537 A1 | 01-07-2021 |
| | | | CA | 3122070 A1 | 11-06-2020 |
| | | | EP | 3891755 A1 | 13-10-2021 |
| | | | US | 2020176098 A1 | 04-06-2020 |
| | | | US | 2021210184 A1 | 08-07-2021 |
| | | | WO | 2020117869 A1 | 11-06-2020 |
| US 9984772 | B2 | 29-05-2018 | CN | 107292086 A | 24-10-2017 |
| | | | EP | 3229157 A1 | 11-10-2017 |
| | | | US | 2017293725 A1 | 12-10-2017 |
| US 10796102 | B2 | 06-10-2020 | CN | 110612525 A | 24-12-2019 |
| | | | EP | 3622412 A1 | 18-03-2020 |
| | | | JP | 7086993 B2 | 20-06-2022 |
| | | | JP | 2020522044 A | 27-07-2020 |
| | | | JP | 2022050439 A | 30-03-2022 |
| | | | JP | 2022122999 A | 23-08-2022 |
| | | | US | 2018329879 A1 | 15-11-2018 |
| | | | US | 2018329880 A1 | 15-11-2018 |
| | | | US | 2020380214 A1 | 03-12-2020 |
| | | | US | 2021049329 A1 | 18-02-2021 |
| | | | WO | 2018208979 A1 | 15-11-2018 |

EPO FORM P0459

For more details about this annex : see Official Journal of the European Patent Office, No. 12/82